# EUROPÄISCHE PATENTSCHRIFT

(11) **EP 1 750 649 B1**
(45) Veröffentlichungstag und Bekanntmachung des Hinweises auf die Patenterteilung: **28.01.2015**
(21) Anmeldenummer: 05740914.6
(22) Anmeldetag: 25.05.2005
(51) Int. Cl.: A61K 6/083, A61K 6/027, C03C 3/066, C03C 3/064, C03C 3/078, C03C 3/089, C03C 3/091, C03C 3/093, C03C 3/095, C03C 3/097, C03C 3/11, C03C 3/118, C03C 3/17, C03C 3/19, C03C 12/00

(54) **GLUSZUSAMMENSETZUNGEN ALS ANTIMIKROBIELLER ZUSATZ FÜR DENTALMATERIALIEN**
GLASS COMPOSITIONS AS AN ANTIMICROBIAL ADDITIVE FOR DENTAL MATERIALS
COMPOSITIONS DE VERRE SERVANTS D'ADDITIFS ANTIMICROBIENS POUR DES MATERIAUX DENTAIRES

(30) Priorität: 29.05.2004 DE 102004026432
(43) Veröffentlichungstag der Anmeldung: 14.02.2007
(73) Patentinhaber: Schott AG, 55122 Mainz (DE)
(72) Erfinder: KESSLER, Susanne, 84030 Ergolding (DE); FECHNER, Jörg, Hinrich, 55118 Mainz (DE); SENESCHAL, Karine, 55131 Mainz (DE); ZIMMER, José, 55218 Ingelheim (DE)
(74) Vertreter: Sawodny, Michael-Wolfgang
(86) Internationale Anmeldenummer: PCT/EP2005/005632
(87) Internationale Veröffentlichungsnummer: WO 2005/115305

(56) Entgegenhaltungen:
- EP-A- 0 997 132
- WO-A-02/072038
- WO-A-03/018498
- WO-A-2004/076369
- DE-A1- 2 159 915
- DE-A1- 2 347 591
- GB-A- 2 386 121
- US-A- 4 358 549
- DATABASE WPI Section Ch, Week 199051 Derwent Publications Ltd., London, GB; Class D21, AN 1990-380320 XP002345016 & JP 02 275731 A (SOGO SHIKA IRYO KENKYUSH) 9. November 1990 (1990-11-09)
- MULLIGAN A M ET AL: "The effect of increasing copper content in phosphate-based glasses on biofilms of Streptococcus sanguis" BIOMATERIALS, ELSEVIER SCIENCE PUBLISHERS BV., BARKING, GB, Bd. 24, Nr. 10, Mai 2003 (2003-05), Seiten 1797-1807, XP004410238 ISSN: 0142-9612
- BELLANTONE MARIA; WILLIAMS HUW D; HENCH LARRY L: "Broad-spectrum bactericidal activity of Ag2O-doped bioactive glass" ANTIMICROBIAL AGENTS AND CHEMOTHERAPY, Bd. 46, Nr. 6, Juni 2002 (2002-06), Seiten 1940-1945, XP008052320
- BELLANTONE M ; COLEMAN N J ; HENCH LARRY L: "Novel sol-gel derived bioactive glass featuring antibacterial properties" KEY ENGINEERING MATERIALS; 13TH INTERNATIONAL SYMPOSIUM ON CERAMICS IN MEDICINE (BIOCERAMICS) 22-26 NOVEMBER 2000, Bd. 192-195, 2001, Seiten 597-600, XP008052336 Bologna, Italy

## Beschreibung

Die Erfindung betrifft die Verwendung antimikrobieller Zusätze für Materialien zur Zahnrestauration, beispielsweise antimikrobielle Zusätze für Dentalgläser sowie antimikrobieller Materialien zur Zahnrestauration, sogenannte antimikrobielle Dentalgläser. Die Materialien zur Zahnrestauration umfassen Materialien zur Zahnfüllung, wobei die Materialien zur Zahnfüllung Glasionomerzement oder Kompomer umfassen. Des Weiteren werden unter Materialien zur Zahnrestauration auch Zusätze, insbesondere antimikrobielle Zusätze, in Beschichtungs- oder Verblendmaterialien für keramische Dentalstrukturen sowie Dentalgläser verstanden. Dentalgläser sind beispielsweise in der DE 4323143 C1 offenbart, deren Offenbarungsgehalt vollumfänglich in den der vorliegenden Anmeldung mitaufgenommen wird.

Bei diesen antimikrobiellen Zusätzen handelt es sich um antimikrobielle und/oder desinfizierende Glaszusammensetzungen oder Glaskeramiken.

Die Glaszusammensetzungen werden bevorzugt als Pulver, Fasern, Flakes oder Kugeln zugegeben.

Die Verwendung derartiger antimikrobieller Zusätze findet im Bereich der Materialien zur Zahnfüllung statt.

Die Materialien zur Zahnfüllung sind nach Journal de l'Association dentaire canadienne, Okt. 1999, Vol. 65, N° 9, p 500-504 in drei Klassen eingeteilt, Glasionomerzemente, Komposite und Kompomere aber nicht auf diese Klassen beschränkt. Dem Fachmann sind weitere Zahlfüllmaterialien bekannt, die hier ebenfalls eingesetzt werden können.

Gemäß Journal de l'Association dentaire canadienne, Okt. 1999, Vol. 65, N° 9, S. 500-504 vereinen Komposite als Materialien zur Zahnfüllung zwei unterschiedliche Materialien, die gemeinsam, beispielsweise als Mischung, Eigenschaften entwickeln, die jedes Material für sich alleine nicht besitzt. Komposite, wie sie aus dem Stand der Technik bekannt sind, umfassen eine Harz-Matrix und verschiedene anorganische Füllstoffe.

Die Harz-Matrix eines Komposites besteht aus einer Mischung unterschiedlicher Monomere, die je nach Mengenverhältnis in Verbindung mit Art und Mischung der Füllstoffe unterschiedliche Eigenschaften- bzw. Eigenschaftsabstufungen ergeben.

Die Harz-Matrix besteht in der Hauptsache aus den Acrylat-Monomeren PMMA (Polymethylmethacrylat), TEGDMA (Triethylenglycoldimethacrylat) und BIS-GMA (Bisphenol Glycidylmethacrylate Komposit). Derartige Harzsysteme sind oft lichtaushärtbar. Weitere Bestandteile der Harz-Matrix sind häufig Beschleunigter, Verzögerer, Stabilisatoren, Initiatoren. Es sind auch chemisch aushärtbar Systeme bekannt.

Als Füllstoffe eines Komposites finden hauptsächlich Gläser, (Glas)-Keramik, Quarz, Sol-Gel-Materialien und Aerosile Verwendung.

Der Füllstoff wird in die Matrix eingebettet, um das physikalische und chemische Verhalten des Verbunds, also des Komposites, zu steuern. Die Füllstoffe verbessern insbesondere die Polymerisationsschrumpfung und verbessern beispielsweise die mechanischen Eigenschaften, wie E-Modul, Biegefestigkeit, Härte und Abrasionsfestigkeit.

Die Aushärtung des Materials erfolgt durch chemische Reaktionen, angestoßen durch das Mischen verschiedener Komponenten, Licht oder Wärme. Unter dem Einfluss von Licht, beispielsweise Licht einer UV-Lampe, einer Halogen-Lampe, einer Plasma-Lampe oder einer LED-Lampe (Licht emittierenden Diode), insbesondere einer LED, die Wellenlängen im Blauen emittiert, und im Zusammenspiel mit Zusatzstoffen werden reaktive Radikale gebildet. Diese Radikale setzen beispielsweise eine Kettenreaktion in Gang, bei der die Monomere des Matrixmaterials, z.B Bis-GMA, über ein radikalisches Zwischenprodukt zu immer längeren Kettenmolekülen zusammengesetzt werden und der Kunststoff so aushärtet. Bei dem Prozess handelt es sich also um eine "radikalische Polymerisation". Bei der radikalischen Polymerisation lagert sich das Zwischenprodukt beispielsweise an die Kohlenstoffdoppelbindung eines weiteren Monomers an. Dadurch entsteht wieder ein Radikal usw., so dass eine Kettenreaktion eintritt.

Weiterhin ist es bevorzugt, dass die Füllstoffe des Komposites nicht erkennbar sind, was eine möglichst gute Anpassung der Brechnungsindexe des ausgehärteten Harzes und des Füllstoffe's erfordert. Vorteilhaft ist auch eine möglichst kleine Korngröße des Füllstoffes, die wiederum die Polierbarkeit der gesamten Füllung, d.h. des Komposites, verbessert. Dafür sind die Partikel mit Korngrössen kleiner als 100 µm, bevorzugt kleiner 50 µm, ganz besonders bevorzugt kleiner 10 µm, geeignet. Unterschreitet die Partikelgrösse einen Wert kleiner als 2 nm, bevorzugt kleiner als 5 nm, besonders bevorzugt kleiner als 10 nm, so sind die mechanischen Eigenschaften der Komposite zu schwach.

Bei den Füllstoffen ist es auch möglich, Mischungen von Partikeln unterschiedlicher Grösse zu benützen, beispielsweise ein Pulver mit einer mittleren Korngrösse im nm-Bereich und ein Pulver mit einer mittleren Korngrösse im Bereich von µm. Mit einer solchen Mischung können die Polierbarkeit und die mechanischen Eigenschaften des Komposites erhöht werden.

Die Komposite gemäß dem Stand der Technik weisen eine geringe Polymerisationsschrumpfung auf. Ist die Polymersiationsschrumpfung zu hoch, so würden hohe Spannungen zwischen Zahnwand und Füllung auftreten. Bei zu großer Polymeristionsschrumpfung kann im Extremfall sogar eine Zahnwand brechen. Ist die Adhäsion zwischen Füllung und Zahnwand schlecht und/oder schrumpft das Material zur Zahnfüllung zu stark, so kann es zur Bildung von Randspalten kommen, was in der Folge wieder zu Sekundärkaries führen kann. Zur Zeit erhältliche handelsübliche Materialien schrumpfen um ca. 1,5-2%.

Insbesondere für Anwendungen im Frontzahnbereich weisen die Komposite, eine Farbe und Transluzenz auf, so dass der Komposit nicht von der umgebenden gesunden Zahnsubstanz unterscheidbar ist. Deshalb ist das Material im wesentlichen farblich an die gesunde Zahnsubstanz angepasst und die Transluzenz entspricht im wesentlichen der eines natürlichen Zahns.

Betreffend die mechanischen Eigenschaften ist es vorteilhaft, wenn die bruchmechanischen Eigenschaften so sind, dass die Füllung bei Kauvorgängen nicht zu stark verschleißt und andererseits der gegenüberliegende Zahn nicht geschädigt wird.

Betreffend die thermische Ausdehnung des Komposites ist es vorteilhaft, wenn diese weitgehend der thermischen Ausdehnung der Zahnsubstanz angepasst ist.

Betreffend die chemische Beständigkeit des Komposites ist dieser so ausgebildet, dass der Komposit gegen basische Angriffe eine ausreichende Stabilität besitzt.

Des Weiteren weist der Komposit, eine Röntgenopazität auf, so dass die Füllung im Röntgenbild vom gesunden Zahn und etwaigem Sekundärkaries unterscheidbar ist.

Betreffend die Rheologie ist das Harz vorteilhafterweise thixotrop, d.h. unter Ausübung von Druck nimmt die Viskosität ab, danach wieder zu. Dieses Verhalten ist deswegen von Vorteil, da das Harz aus Kartuschen in die Cavität eingefüllt werden muss, andererseits aber auch vor der Aushärtung möglichst formstabil sein muss.

Der Begriff Glasionomerzement ist in der ISO 7484 definiert, deren Offenbarungsgehalt vollumfänglich in die vorliegende Anmeldung mitaufgenommen wird.

Als Gtasionomerzement sind bespielsweise wässerige Poly-(Carbonsäure)-Zement-Zusammensetzungen bekannt und werden bereits in der Zahnheilkunde eingesetzt. Glasionomerzemente umfassen ein Polymer, das freie Carbonsäuregruppen, typischerweise ein Homo- oder Co-Polymer einer Acrylsäure, enthält und ein Ionen freisetzendes Glas, wie zum Beispiel ein Calcium-Aluminiumfluorosilicatglas.

Glasionomerzemente bilden sich über eine Säure-Basen-Reaktion in wässeriger Lösung. In Gegenwart von Wasser setzt das Glas polyvalente Metallionen, wie zum Beispiel Aluminium- und Calcium-Ionen, frei. Diese dienen zur Vernetzung des Polymers. So wird eine starre gelatineartige Struktur erhalten. Zur gleichen Zeit reagiert das Material im Glas mit Wasser und bildet Kieselsäure. Als Ergebnis dieser Gel bildenden Reaktion bildet sich ein Zement, der für Dentalanwendungen geeignet ist.

Da Glasionomerzemente spröde und wenig elastisch sind, ist deren Verwendung aufgrund der unzureichenden mechanischen Eigenschaften sehr beschränkt. Um die mechanischen Eigenschaften von Glasionomerzementen zu verbessern, ist es beispielsweise bekannt, die Matrix zu modifizieren. Hierzu wurden entweder ungesättigte Kohlenstoff-Kohlenstoff-Bindungen auf ein Polyalkanoat-Grundgerüst aufgepfropft oder (Di)Methacrylatmonomer(e) in die Zusammensetzung einbezogen oder beides wurde durchgeführt. Ungesättigte Kohlenstoff-Kohlenstoff-Bindungen ermöglichen eine kovalente Vernetzung der Matrix über eine radiaklische Polymerisation (chemisch oder durch Lichtstrahlen). Eine kovalent vernetzte Matrix verbessert deutlich die mechanischen Eigenschaften des abgebundenen Zements. Dieser Zement wird vom Zahnmark gut vertragen. Jedoch traten Probleme hinsichtlich der Biokompatibilität auf, da unerwünschter Weise Harzkomponenten freigesetzt werden können, wie Hydroxyethylmethacrylat oder HEMA. Diese Verbindungen sind als Harz-modifizierte Glasionomerzemente (resin-modified glass ionomer cements, RMGICs) bekannt, obwohl ihre Struktur besser als Harz-modifizierte Glas-Polyalkanoat-Zemente beschrieben werden würde. Diese RMGICs sind auf Wasserbasis, eine Säure-Base-Reaktion ist der Hauptabbindemechanismus und sie behalten dabei ihre Fähigkeit über die Carboxylgruppen der Polyalkanoat-Komponente an hartes Zahngewebe zu binden. Ihre Fluorid-Freisetzung ist ähnlich zu den GICs.

Weiterhin bekannt sind polymerisierbare Zemente, wie sie zum Beispiel in EP-A-0219058 beschrieben werden und unter der Bezeichnung "Kompomer" und "kunststoffverstärkter Glasionomerzement" bekannt sind.

Bei dem kunststoffverstärkten Glasionomerzement-Kompomer handelt es sich um ein Material, das die Vorzüge eines Kompositwerkstoffes (die Silbe "Komp" im Namen) und die eines Glasionomeren (die Silbe "omer" im Namen) miteinander vereint. Das Material umfasst Dimethylmetacrylat-Monomere mit zwei Carboxylgruppen und ein Füllmaterial, das im wesentlichen ein ionenabgebendes Glas ist. Das Verhältnis der Carboxylgruppen zu den Kohlenstoffatomen des Rückgrates beträgt 1:8. Die Zusammensetzung ist wasserfrei und das ionenabgebende Glas ist teilweise silanisiert, um eine Bindung mit der Matrix sicherzustellen. Diese als Kompomer bezeichneten Materialien binden über eine radikalische Polymerisation ab, können aber nicht an hartes Zahngewebe binden und weisen eine beträchtlich niedrigere Fluorid-Freisetzung als Glasionomerzemente auf.

Sie haben einen niedrigeren elastischen Biegemodul, eine niedrige Biege-, Druck- und Bruchfestigkeit und geringe Härte. Die Kompomere sind einsetzbar als Kleber in der Kieferorthopädie, als Amalgam-Bonding-System und im Bereich der Veterinärmedizin. Da diese Materialien nicht über eine Säure-Base-Reaktion abbinden und auch nicht an hartes Zahngewebe binden, sollten diese eigentlich nicht als Glasionomerzemente klassifiziert werden, da sie ein völlig anderes Material darstellen.

Ferner werden Kompomere häufig nicht ganz richtig als "hybride Glasionomeren", "lichtgehärtete. GICs", oder auch "Harz-modifizierte Glasionomere" bezeichnet, d.h. genauso wie die tatsächlichen "Harz-modifizierten Glasionomere". Auch der Begriff "Polysäuremodifizierte Kompositharze" ist gebräuchlich.

Für alle Arten von Materialien zur Zahnfüllung, wie insbesondere Glasionomere, Komposite und Kompomere gilt, dass sie als Füll- oder Zuschlagstoffe neben den inerten oder reaktiven Dentalgläsern als weitere Füllstoffe Aerosile, bspw. pyrogene Kieselsäure enthalten können, die zur Einstellung der Rheologie eingesetzt werden. Die Aerosile haben im Gegensatz zu den gemahlenen Glaspulvern sphärische Form und Partikelgrößen von ca. 50 - 300 nm.

Als weitere Füllstoffe können Pigmente zur Einstellung der Zahnfarben enthalten sein, sowie Stoffe zum Erreichen der Röntgenopazität. Derartige Stoffe sind beispielsweise BaSO₄, ZrO₂, YbF₃.

Auch Sol-Gel-Materialien, wie z. B. Zr-Silikate, die Röntgenopazität aufweisen, sind als Füllmaterial denkbar.

Des Weiteren können organische Fluoreszenzfarbstoffe zur Nachbildung der Fluoreszenzeigenschaften des natürlichen Zahnes vorgesehen sein.

Nachteilig an den bekannten Materialien im Bereich der Zahnheilkunde, insbesondere den Glasionomerzementen, den Kompositen und den Kompomeren war, dass sie keine antimikrobielle Wirkung aufweisen und somit vor antimikrobiell ausgelösten Zahnerkrankungen wie beispielsweise Sekundärkaries, Wurzelentzündungen oder Parodontose nicht ausreichend schützen.

Die antimikrobielle, entzündungshemmende und wundheilende Wirkung von Gläsern, insbesondere hieraus hergestellten Glaspulvern ist aus nachfolgenden Schriften bekannt geworden:
WO 03/018496
WO 03/018498
WO 03/018499

Die WO03/018496 und die WO03/018499 zeigen ein entzündungshemmendes und wundheilendes Silicatglaspulver.

Aus der WO 03/018498 ist antimikrobielles, entzündungshemmendes Glas und Glaspulver bekannt geworden, das in der Glaszusammensetzung mehr als 10 ppm lod enthält. Aus WO 02/072038 und EP-A-1365727,
ist die Verwendung von Alkali-Erdalkaligläsern ohne Ag, Zn, Cu in Dentalmaterialien bekannt.

Die WO 2004/076369 offenbart antimikrobiell wirkende Borosilicatgläser mit der nachfolgenden Zusammensetzung in Gew. -% auf Oxidbasis: SiO₂40 - 80 Gew.-%, B₂O₃ 5 - 40 Gew.-%, Al₂O₃ 0 - 10 Gew.-%, P₂O₅ 0 - 30 Gew.-%, Li₂O 0 - 25 Gew.-%, Na₂O 0 - 25 Gew.-%, K₂O 0 - 25 Gew.-%, CaO 0 - 25 Gew.-%, MgO 0 - 15 Gew.-%, SrO 0 - 15 Gew.-%, BaO 0 - 15 Gew.-%, ZnO 0 - < 15 Gew.-%, Ag₂O 0,01 - 5 Gew.-%, CuO 0 - 10 Gew.-%, GeO₂ 0 - 10 Gew.-%, TeO₂ 0 -15 Gew.-%, Cr₂O₃ 0 - 10 Gew.-%, J 0 - 10 Gew.-%, F 0 - 10 Gew.-%, wobei die Summe ZnO + Ag₂O + CuO + GeO₂ + TeO₂ + Cr₂O₃ + J zwischen 5 und 70 Gew.-% liegt. Die Gläser sind zur Verwendung für Dentalmaterialien, insbesondere für Zahnfüllungen, Kronen, Inlets geeignet, wobei auch die Verwendung als Compositwerkstoff mit Polymerwerkstoffen beschreiben wird.

Aufgabe der Erfindung ist es, die Nachteile des Standes der Technik zu überwinden und insbesondere Zusätze für Dentalmaterialien bereitzustellen, die eine antimikrobielle und desinfizierende, entzündungshemmende und wundheilende Wirkung besitzen.

Gelöst wird die Aufgabe gemäß den unabhängigen Ansprüchen. Vorteilhafte Weiterbildungen sind Gegenstand der Unteransprüche.

In einer besonders bevorzugten Ausführungsform fungieren die antimikrobiellen Zusätze, die im folgenden auch als antimikrobielle Dentalglaspulver bezeichnet werden, selbst als Glasionomere, d.h. sie besitzen neben der antimikrobiellen Wirkung noch die Funktion als Initiator für eine Polymerisation von Monomeren zu dienen bzw. stellen die für die Aushärtreaktion zu einem Glasionomerzement notwendigen Ionen, bspw. die Ca²⁺-, Al³⁺-Ionen,zur Verfügung. Beispielsweise bewirkt die Auslaugung von Ca²⁺-, Al³+ -Ionen zusammen mit z. B. den Polycarbonsäuren der Kunststoffe des Zementes die Aushärtung. Es handelt sich also in diesem Fall um reaktive antimikrobielle Dentalglaspulver.

In einer alternativen Ausführungsform hat das antimikrobielle Glas selbst keine lonomereneigenschaften, sondern fungiert als Zuschlagmaterial, das die antimikrobielle Wirkung zur Verfügung stellt. Es handelt sich also um ein inertes antimikrobielles Dentalglaspulver. Wird das antimikrobielle Dentalglaspulver lediglich als Zuschlagstoff, also als inertes antimikrobielles Dentalglaspulver eingesetzt, so kann die Polymerisation der Monomere beispielsweise durch Licht z. B. UV-Strahlung oder Wärme erreicht werden.

In einer weitergebildeten Ausführungsform ist das inerte oder auch das reaktive antimikrobielle Dentalglaspulver derart ausgestaltet, dass der Schrumpf des sich nach der Polymersiation ergebenden Glasionomerzementes oder Kompomeres verringert oder Röntgenopatizität erzielt wird. Auch die Ausgestaltung des antimikrobiellen Dentalglaspulvers derart, dass eine Remineralisierung des Zahnschmelzes unterstützt wird, ist möglich.

Selbstverständlich sind auch Mischungen des erfindungsgemäßen antimikrobiellen Dentalglaspulvers mit anderen Dentalfüllern, beispielsweise herkömmlichen Dentalgläsern möglich.

In einer bevorzugten Ausführungsform der Erfindung ist der thermische Ausdehnungskoeffizient, die CTE des antimikrobiellen Dentalglaspulvers sehr klein und liegt zwischen 3·10 ⁻⁶ / K und 8·10⁻⁶/K.

Bevorzugt ist der Brechungsindex des antimikrobiellen Dentalglaspulvers so gewählt, dass der Brechungsindex weitgehend an den der Matrix angepasst ist, wobei das Glaspulver selbst weitgehend frei von färbenden Ionen ist.

In einer weitergebildeten Ausführungsform ist die Glaspulveroberfläche des antimikrobiellen Dentalglaspulvers silanisiert, so dass ein chemischer Verbund zwischen Füllstoffpartikel und Harzmatrix ermöglicht wird. Dies wiederum hat verbesserte mechanische und rheologische Eigenschaften der Füllung bzw. der Formulierung zur Folge.

Besonders bevorzugt ist es, wenn das antimikrobielle Dentalglaspulver eine gute chemische und hydrolytische Beständigkeit sowie eine hohe Röntgenopazität (RO) aufweist.

Eine hohe Röntgenopazität wird insbesondere durch Zugabe von schweren Elementen, wie Sr oder Ba, erreicht.

Um die Ästhetik und die Polierbarkeit zu verbessern, sind kleine Korngrößen des antimikrobiellen Dentalglaspulvers von d50 zwischen 0,4 - 5 µm bevorzugt.

Besonders bevorzugt sind Ausführungsformen, die antimikrobielle Langzeitwirkung besitzen.

Besonders bevorzugt besitzen die Materialien eine hohe antimikrobielle und desinfizierende Wirkung, setzen aber keine bzw. nur sehr geringe Mengen antimikrobieller Ionen wie zum Beispiel Zink oder Silber frei.

Bevorzugt findet das erfindungsgemäße antimikrobielle Glas Verwendung in Beschichtungs-, Füll- oder Verblendmaterialien für die Zahnheilkunde.

Im Gegensatz zu Implantatmaterialien, die in den Kiefer eingebracht werden, werden die in dieser Anmeldung beschriebenen Materialien bevorzugt im bzw. am Zahn verwendet.

In einer besonderen Anwendung in Glasionomerzementen umfassen die Zemente den antimikrobiellen Glaszusatz oder die antimikrobielle Glaskeramik in einer Konzentration im Bereich von 0,01 - 99,5 Gew%. Bevorzugt sind 0,1 bis 80 Gew%, insbesondere bevorzugt sind 1 bis 20 Gew% antimikrobieller Glaszusatz oder Glaskeramikzusatz in Glasionomerzementen enthalten.

Die antimikrobiell wirkenden Gläser, verwendet gemäß der Erfindung, können auch mit bekannten Glaspulvern, die als Dentalfüllmaterialien eingesetzt werden, gemischt werden.

Die Partikelgrösse der antimikrobiellen Glaspulver ist beispielsweise bei d50-Werten grösser als 0,1 um, bevorzugt grösser als 0,5 µm, noch bevorzugter grösser als 1µm.

Die Partikelgrösse der antimikrobiellen Glaspulver ist bei d50-Werten beispielsweise kleiner als 200 µm, bevorzugt kleiner als 100 µm, noch bevorzugter kleiner als 20 µm. Am bevorzugtesten sind Partikelgrössenverteilungen mit Partikelgrössen größer als 0,1 µm und kleiner gleich 10 µm, insbesondere wegen der besseren Polierbarkeit zwischen 0, 1 - 1,5 µm.

Die Gläser enthalten in bevorzugten Ausführungsbeispielen antimikrobiell wirksame Elemente bzw. Ionen, wie z. B. Ag, Zn, Cu. Die Freisetzungsraten der antimikrobiell wirkenden Ionen sind in den Glasmatrizen so gering, dass kein Gesundheitsrisiko besteht, andererseits aber eine hinreichende antimikrobielle Wirkung erzielt wird.

Beispielsweise wird bei der Freisetzung von Silber als antimikrobiellem Ion eine hinreichende Freisetzung für eine antimikrobielle Wirkung erreicht, die noch nicht zu gesundheitlichen Schäden führt, wenn die Freisetzungsraten von z. B. Silber in Wasser aus den erfindungsgemäßen Gläsern unterhalb von 1000 mg/l bevorzugt < 500 mg/l und bevorzugter < 100mg/l liegt. Besonders bevorzugt liegen diese < 50 mg/l und bevorzugter < 20mg/l. In einer ganz besonders bevorzugten Ausführungsform liegen diese < 10mg/l.

Ist das antimikrobielle Glas in ein Kompositmaterial eingebracht, so werden in Kontakt mit Flüssigkeit wie z. B. Wasser oder Mundspeichel, noch geringere Mengen Silber freigesetzt, als aus dem freien Glas in Wasser. Freisetzungsraten von z. B. Silber in Wasser aus dem erfindungsgemäßen Glassionomerzement oder Kompomer liegen beispielsweise unterhalb von 10 mg/l, bevorzugt < 1 mg/l ganz besonders bevorzugt < 0,1 mg/L.

Um eine ausreichende antimikrobielle Wirkung zur Verfügung zu stellen, liegen die Freisetzungsraten beispielsweise für Ag oberhalb von 0,0001 mg/l, bevorzugt oberhalb von 0,001 mg/l und ganz besonders bevorzugt oberhalb von 0,01 mg/l. Als Basisgläser kommen Phosphat-, Borat und Silicat-Gläser in Frage, die keine zu hohe chemische Beständigkeit aufweisen.

Vorteilhaft ist, dass diese Gläser in ihrer Brechzahl anpassbar sind.

Um eine antimikrobielle und desinfizierende Wirkung zu erhalten, ist der Gehalt von Ionen, wie bspw. Ag, Zn, Cu, im Glasionomer größer als 0,01 Gew%, bevorzugt größer als 0,1 Gew%, noch bevorzugter größer als 0,5 Gew%. Bevorzugt sind in Abgrenzung zur WO 93/17653A1 weniger als 30 Atom-% Zn in der Glaszusammensetzung enthalten.

Besteht in einer bevorzugten Ausführungsform eine erfindungsgemäße Mischung aus einem antimikrobiellen Glaspulver, das auch in dieser Anmeldung als antimikrobielles Dentalglaspulver bezeichnet wird, und einem Glasionomer und/oder einem Dentalglasfüller, so ist das Verhältnis von antimikrobiellem Glaspulver / Glasionomer und/oder Dentalglasfüller > 0,0001, bevorzugt größer als 0,001, ganz besonders bevorzugt größer als 0,01.

Ist der Gehalt an antimikrobiellem Glaspulver zu niedrig, d.h. ist das Verhältnis antimikrobielles Glaspulver / Glasionomer und/oder Dentalglasfüller < 0,0001, so wird keine ausreichende antimikrobielle und desinfizierende Wirkung der Mischung mehr erzielt.

Bevorzugt ist das Verhältnis von antimikrobiellem Glaspulver / Glasionomer und/oder Dentalglasfüller < 200, bevorzugt < als 100, ganz besonders bevorzugt < als 10.

Weist die Mischung ein Verhältnis von antimokrobiellem Glaspulver / Glasionomer und/oder Dentalglasfüller auf, das größer als 200 ist, so wird in der Regel keine ausreichende Initiierung der Polymerisation der Monomere durch das Glasionomer mehr erzielt.

In einer besonderen Ausführungsform stellt das antimikrobielle Pulver, wenn es in Kontakt mit Wasser bspw. Mundspeichel etc. kommt durch Ionenaustausch mit der Glasmatrix einen basischen pH, d.h. einen pH-Wert >7 ein. Dieser neutralisiert Säuren, die durch Kariesbakterien gebildet werden, und den Zahn bzw. den Zahnschmelz angreifen können. Insbesondere verhindert diese Reaktion den Angriff in den Zwischenräumen zwischen dem Dentalmaterial und dem Zahn.

Die Kombination der antimikrobiellen Glaspulver mit besonders remineralisierenden Glaspulvern, wie z. B. einem Glaspulver wie in der EP-A-1365727 offenbart, ist möglich und bevorzugt. Dadurch wird zum einen eine enge Verbindung zwischen Zahn und Dentalmaterial erreicht und zum anderen, da remineralisierende Glaspulver, wie z. B. die Glaspulver aus der EP-A-1365727, ebenfalls eine geringe antimikrobielle Wirkung besitzen, ein antimikrobieller synergistischer Effekt erzielt. In der EP-A-1365727 ist die Verwendung von bioaktivem Glas zur Herstellung eines Mittels für eine permanente Zahnfüllung beschrieben. Das bioaktive Glas ist vorzugsweise in einem Bonding, das als Haftvermittler zwischen Zahnsubstanz und Füllungsmaterial dient, in einem Glasionomerzement, in einem Glas-Kunststoff-Composit, in einem kompositverstärkten Glasionomerzement und/oder in einem Mittel zur Behandlung der Zahnwurzel, des Zahnhalses und/oder der Zahnkrone enthalten und enthält vorzugsweise Fluoridionen.

Eine antimikrobielle Wirkung, beispielsweise durch die Freisetzung von Ag, Zn oder Cu-Ionen, wird in dem Glasionomerzement, in dem Glas-KunststoffKomposit, in dem kompositverstärkten Glasionomerzement und/oder in dem Mittel zur Behandlung der Zahnwurzel, des Zahnhalses und/oder der Zahnkrone, das das bioaktive Glas, das in der EP-A-1365727 beschrieben ist, enthält, nicht beschrieben. Besonders bevorzugt besitzt das Glas eine hohe Röntgenopazität.

In einer bevorzugten Ausführungsform setzt der antimikrobielle Glaszusatz Fluorid frei, wie beispielsweise die Glaszusammensetzung, die in der WO 03/018499 offenbart ist. Die Auswahl eines derartigen antimikrobiellen Glaspulvers beugt der Bildung von Karies vor. Bevorzugt besitzt das antimikrobielle Glaspulver remineralisierende Eigenschaften.

In einer weitergebildeten Ausführungsform fungiert der antimikrobielle Zusatz selbst als Glasionomer, d.h. er stellt die für die Aushärtreaktion zu einem Glasionomerzement notwendigen Ionen, bspw. die Ca²⁺, Al³⁺-Ionen, zur Verfügung. Die Auslaugung von Ca²⁺, Al³⁺-Ionen bewirkt zusammen mit z. B. den Polycarbonsäuren der Kunststoffe die Aushärtung des Zementes. Für die remineralisierenden, Eigenschaften werden Glaszusammensetzungen bevorzugt eingesetzt, die Ca und/oder Phosphorionen und / oder Natrium und/oder Verbindungen, die Ca oder Phosphor enthalten, freisetzen, und so die Remineralisierung der Zähne unterstützen.

Bekannte Glasionomerzemente bestehen häufig aus einem Pulver-Flüssigkeit-System.

Der Glasionomerzement entsteht durch eine Abbindereaktion der flüssigen Komponente mit dem Glasionomeren wie unten beschrieben.

In der Regel werden die organischen Bestandteile zu einer Flüssigkeit verarbeitet, ergeben die flüssige Komponente, die erst direkt vor der Anwendung vom Zahnarzt mit der festen Komponente, insbesondere dem Pulver, insbesondere dem Glaspulver, dem sogenannten Glasionomeren, innig vermischt wird. Die Flüssigkeiten bestehen zum Beispiel aus Polyacrylsäuren, Weinsäure, destilliertem Wasser, Drei-Harz-Komplexen, wie beispielsweise 2-Hydroxyethylmethacrylat (HEMA). Auch üblich sind Paste-Paste-Systeme, bei denen die Bestandteile, die alleine noch keine Reaktion mit dem Glasionomeren bzw. der erfindungsgemäßen Mischung aus Glasionomeren und antimikrobiellem Glaspulver erzielen, mit diesem zu einer Paste vermischt werden, z. B. 2-Hydroxyethylmethacrylat, Dimethacrylate oder Pigmente. Die anderen Bestandteile, wie Polyacrylsäuren, Wasser, Pyrogenkieselsäure, werden in einer zweiten Paste vermischt. Beim Zahnarzt wird dann durch intensives Vermischen der Pasten die Abbindereaktion in Gang gesetzt, die den Glasionomerzement ergibt.

Es sind auch verstärkte Systeme bekannt, in denen zum Beispiel auch Methacrylat-modifizierte Polycarbonsäuren eingesetzt werden.

Soll der Zement dualhärtend ausgestattet sein, ist die Verwendung von Photoinitiatoren, wie zum Beispiel Campherchinon, möglich.

Der Vorteil einer Mischung von antimikrobiell wirkenden Glaspulvern mit nicht antimikrobiell wirksamen Glasionomeren gemäß der Erfindung besteht darin, dass die antimikrobielle Wirkung der Mischung die antmikrobielle Einzelwirkung des Glaspulvers übersteigt, da die Freisetzung antimikrobiell wirksamer Ionen, wie zum Beispiel Ag aus dem antimikrobiellen Glaspulver durch die freigesetzten Ionen aus dem Glasionomer angeregt wird.

Ein weiterer Vorteil besteht darin, dass durch den Zusatz von ionenfreisetzendem antimikrobiellem Pulver die radikalische Polymerisation (initiiert durch z. B. Licht oder Wärme), d.h. der Polymerisationsgrad und somit der Festigkeitsgrad (z. B. E-Modul etc.) sowie die Kinetik der Polymerisation des Zementes, synergistisch unterstützt wird.

Sind bei Kompositen die oben beschriebenen Füllstoffe Glasfüller, die biozide Ionen wie z. B. Ag⁺, Zn²⁺, Cu²⁺ enthalten, so kann durch die Freisetzung dieser Ionen aus dem Glas der gesamte Komposit eine antimikrobielle Wirkung aufweisen. Dadurch, dass der gesamte Komposit eine antimikrobielle Wirkung aufweist, wird die Bildung von Sekundärkaries vermieden, zumindest aber deutlich verlangsamt.

Die als Füllstoff verwendeten Glasfüller können selbst keine antimikrobielle Wirkung aufweisen, aber Teil einer Mischung aus Glasfüller und antimikrobiellem Glaspulver sein.

Bei den Glasionomerzementen ist es auch durch den Zusatz von antimikrobiellen Gläsern möglich, dass die carboxylhaltigen Gruppen der Polyalkenoatketten das Kalzium der Hydroxylapatitschicht des antimikrobiellen Glaspulvers chelieren, um den Kleber zu mineralisiertem Hartzahngeweben abzubinden. Durch die Zugabe von antimikrobiellem Glaspulver in einen Glasionomerzement ist es also möglich, dass ein fester Verbund zur Zahnhartsubstanz entsteht.

Darüber hinaus induzieren die Ionen der Reaktion, die zur Einstellung der Glasionomerzement verwendet werden, dass Kalzium-, Aluminium-, Natrium-, Fluorid- und Kieselsäureionen vom säurelöslichen Glas freigegeben werden.

Unter einem strukturellen Gesichtspunkt ist ein Glasionomerzement ein Komposit, in dem die unreagierten Glaspartikel Matrialfüller sind und die Kalzium-Aluminium querverbundenen Polyalkenoatketten die Matrix bilden. Die von der Matrix umschlossenen Glaspartikel stellen dann eine Bindung zwischen dem Füller und der Matrix dar.

Die ionischen Bindungen sind für die Vernetzung der Polymerketten und das Abbinden des Glasionomerzementes verantwortlich. Die große Anzahl sekundärer Bindungen spielen eine wichtige Rolle bei der Einstellung der mechanischen Eigenschaften des Zementes.

Glasionomerzemente sind spröde und haben einen niedrigen Elastizitätsmodus, sie sind unter Zugspannung schwach und haben eine niedrige Bruchfestigkeit. Wegen Ihrer schlechten mechanischen Eigenschaften ist ihre Verwendung als Zahnrestaurationsmaterial beschränkt.

Eine Möglichkeit, die mechanischen Eigenschaften von Glasionomerzementen zu verbessern, besteht in einer verbesserten Matrix. Hier wurden Fortschritte gegenüber dem Stand der Technik erzielt, indem man zur Verstärkung der Matrix antimikrobielle Gläser einsetzt, was zu einer festen Bindung an das Hartzahngewebe führt.

Bei Kompomeren wird durch die Zugabe antimikrobieller Glaspulver der Vorteil erzielt, dass der Schrumpf geringer wird. Des Weiteren werden die mechanischen Eigenschaften von Glasionomeren verbessert und ein starker Bindungseffekt der Komposite erzielt.

Nachfolgend soll die Erfindung anhand von Ausführungsbeispielen erläutert werden ohne hierauf beschränkt zu sein.

Als antimikrobieller Glaszusatz zu einem Glasionomer in einem Glasionomerzement, insbesondere in Form eines antimikrobiellen Glaspulvers sind bespielsweise Borosilicatgläser geeignet. Zunächst sollen Ausführungsbeispiele für Borosilicatgrundgläser angegeben werden, die keiner besonderen Behandlung zur Erzielung eines phasenentmischten Systems unterzogen wurden.

Die Gläser wurden dadurch erhalten, dass aus den Rohstoffen ein Glas erschmolzen wurde, das anschließend zu Ribbons geformt wurde. Diese Ribbons wurden mittels Trockenmahlung zu Pulver mit einer Partikelgröße d50 = 4 µm weiterverarbeitet.

In Tabelle 1 sind Glaszusammensetzungen in Gew% auf Oxidbasis erfindungsgemäßer Borosilicatgläser angegeben, die zu einem Glaspulver gemahlen werden können und in dem Glasionomerzement Verwendung finden.

Die Zusammensetzungen A1, A5, A13, A14, A15, A46, A51, A53, A54, A61, A68 gehören nicht zu der Erfindung.

**Tabelle 1:**

| Zusammensetzungen in Gew% auf Oxidbasis von erfindungsgemäßen Borosilicatgläsern | | | | | | | | | | | | | | | | | |
|---|---|---|---|---|---|---|---|---|---|---|---|---|---|---|---|---|---|
| | A1 | A2 | A3 | A4 | A5 | A6 | A7 | A8 | A9 | A10 | A11 | A12 | A13 | A14 | A15 | A16 | A17 |
| SiO₂ | 63,5 | 63,5 | 62,5 | 71 | 61 | 69 | 61 | 61 | 64,5 | 60,99 | 56,2 | 63,5 | 77 | 70 | 57 | 63,5 | 61 |
| B₂O₃ | 30 | 29,9 | 28 | 21 | 21 | 16 | 22 | 36 | 25,5 | 22 | 18 | 29 | 14,5 , | 10,7 | 27 | 29 | 37 |
| Al₂O₃ | | | 4 | | | 2,75 | | | | | 6,63 | 6,63 | 4 | 4 | | | |
| P₂O₅ | | | | | | 2,75 | | | | | | | | | | | |
| Na₂O | 6,5 | 6,5 | | 7 | | 6 | 3 | 2,99 | 4,7 | 5 | 3,7 | 6,5 | 3,5 | 2,8 | 6 | 5.5 | |
| Li₂O | | | | | | | | | | | 1,84 | | | | | | |
| K₂O | | | | | | | 4 | | | | 5,64 | | 1 | 3,6 | | | |
| BaO | | | 5 | | | | | | | | | | | | | | |
| CaO | | | | | | 3 | | | | | | | | 2,1 | | | |
| MgO | | | | | | | | | | | | | | | | | |
| SrO | | | | | | | | | | | | | | | | | |
| ZnO | | | | | 18 | | 9,95 | | | | 0,28 | | | 2,5 | 10 | | |
| SO₃ | | | | | | | | | | | 5,37 | | | | | | |
| Ag₂O | | 0,1 | 0,5 | 1 | | 0,5 | 0,05 | 0,01 | 5 | 0,01 | 0,21 | 1 | | | | 2 | 2 |
| CuO | | | | | | | | | | 2 | 2,07 | | | | | | |
| GeO₂ | | | | | | | | | | | | | | | | | |
| TeO₂ | | | | | | | | | | 1 | 0,04 | | | | | | |
| Cr₂O₃ | | | | | | | | | | 1 | 0,01 | | | | | | |
| ZrO₂ | | | | | | | | | | | | | | 4,3 | | | |
| Jod | | | | | | | | | | | 0,01 | | | | | | |
| Br | | | | | | | | | | | | | | | | | |
| Cl | | | | | | | | | | | | | | | | | |
| La₂O₃ | | | | | | | | | 0,3 | | | | | | | | |

In Tabelle 2 sind Borosilicatgläser angegeben, die einem definierten Temperungsprozess unterzogen wurden. Durch diese Temperung wurde eine definierte Entmischung in Mehrphasensysteme, insbesondere ein 2-PhasenSystem erreicht. Die Gläser wurden aus den Rohstoffen wie für die jeweiligen Ausführungsbeispiele in Tabelle 1 angegeben erschmolzen und anschließend zu Ribbons geformt. Sodann wurde die in Tabelle 2 angegebene Temperung bei den angegebenen Temperaturen für die angegebene Zeit durchgeführt. In Tabelle 2 ist für die unterschiedlichen Glaszusammensetzungen gemäß Tabelle 1 die Tempertemperatur, die Temperzeit sowie die Größe der entmischten Bereiche, bei einem 2-Phasensystem, die sogenannte Entmischungsgröße, angegeben.

**Tabelle 2:**

| Größe der entmischten Bereiche für unterschiedliche Glaszusammensetzungen für unterschiedliche Temperaturen und Temperzeiten | | | | | |
|---|---|---|---|---|---|
| **Probe** | **Glaszusammensetzung gemäß Tabelle 1** | **Temperung an** | **Temperatur (°C)** | **Zeit (h)** | **Entmischungsgröße** |
| Ausf. 1-a | Ausf. 1 | Ribbon | 560 | 10 | 30 nm |
| Ausf. 1-b | Ausf. 1 | Ribbon | 560 | 20 | 60 nm |
| Ausf. 1-c | Ausf. 1 | Ribbon | 620 | 10 | 40 nm |
| Ausf. 1-d | Ausf. 1 | Ribbon | 620 | 20 | 80 nm |
| Ausf. 2-a | Ausf. 2 | Ribbon | 560 | 10 | 40 nm |
| Ausf. 2-b | Ausf. 2 | Ribbon | 560 | 20 | 100 nm |
| Ausf. 2-c | Ausf. 2 | Ribbon | 620 | 10 | 70 nm |
| Ausf. 2-d | Ausf. 2 | Ribbon | 620 | 20 | 150 nm |
| Ausf. 12a | Ausf. 12 | Ribbon | 560 | 10 | 50 nm |
| Ausf. 12b | Ausf. 12 | Ribbon | 560 | 20 | 150 nm |
| Ausf. 12c | Ausf. 12 | Ribbon | 620 | 10 | 80 nm |
| Ausf. 12d | Ausf. 12 | Ribbon | 620 | 20 | 200 nm |
| Ausf. 14a | Ausf. 14 | Ribbon | 820 | 5 | 40 nm |

Bei den Systemen gemäß Tabelle 2 handelt es sich um Zwei-Phasen-Systeme, wobei die Zusammensetzungen der beiden Phasen unterschiedlich sind. Die eine Phase ist eine Phase, in der Bor angereichert ist, die andere Phase ist eine Phase, in der Silicium angereichert ist. Durch die niedrigere chemische Beständigkeit der borreichen Phase, wird die antimikrobielle Wirksamkeit erhöht, da die Abgabe von antimikrobiellen Ionen, wie z. B. Silber, schneller erfolgen kann.

In den Tabellen 3 bis 5 ist für unterschiedliche Ausführungsbeispiele von Glaszusammensetzungen gemäß Tabelle 1 die antimikrobielle Wirkung angegeben. Es handelt sich bei der Ermittlung der antimikrobiellen Wirkung in allen Fällen um Messungen aus den Gläsern der jeweiligen Glaszusammensetzung erhaltenen Glaspulvern, die durch Mahlung aus dem Ribbon erhalten wurden. Eine Temperung am Ribbon wurde lediglich für das in Tabelle 3 angegeben Glaspulver verwendet.

**Tabelle 3:**

| Antibakterielle Wirkung eines Glaspulvers nach Europ. Pharmakopoe (3. Auflage) für eine Glaszusammensetzung gemäß Ausführungsbeispiel 2 in Tabelle 1 mit einer Partikelgröße von 4 µm in einer wässerigen Suspension bei einer Konzentration von 0,01 Gew%. Das Glas wurde vor der Mahlung nicht getempert. | | | | | |
|---|---|---|---|---|---|
| | **E. coii** | **P. aeruginosa** | **S. aureus** | **C. albicans** | **A. niger** |
| **Start** | 350000 | 250000 | 270000 | 333000 | 240000 |
| **2 Tage** | 0 | 0 | < 100 | 0 | 240000 |
| **7 Tage** | 0 | 0 | 0 | 0 | 180000 |
| **14 Tage** | 0 | 0 | 0 | 0 | 50000 |
| **21 Tage** | 0 | 0 | 0 | 0 | 16000 |
| **28 Tage** | 0 | 0 | 0 | 0 | 4000 |

**Tabelle 4:**

| Antibakterielle Wirkung eines Glaspulvers nach Europ. Pharmakopoe (3. Auflage) für eine Glaszusammensetzung gemäß Ausführungsbeispiel 12 mit einer Partikelgröße von 4 µm in einer wässerigen Suspension bei einer Konzentration von 0,001 Gew%. Das Glas wurde vor der Mahlung wie in Ausführungsbeispiel 12c gemäß Tabelle 2 bei 620°C für 10 h am Ribbon getempert, so dass ein in zwei Phasen entmischtes Glas mit einer Entmischungsgröße von 80 nm erhalten wurde. | | | | | |
|---|---|---|---|---|---|
| | **E. coli** | **P. aeruginosa** | **S. aureus** | **C. albicans** | **A. niger** |
| **Start** | 270000 | 260000 | 260000 | 240000 | 240000 |
| **2 Tage** | 0 | 0 | 0 | < 100 | 180000 |
| **7 Tage** | 0 | 0 | 0 | 0 | 100000 |
| **14 Tage** | 0 | 0 | 0 | 0 | 60000 |
| **21 Tage** | 0 | 0 | 0 | 0 | 12000 |
| **28 Tage** | 0 | 0 | 0 | 0 | 6000 |

**Tabelle 5:**

| Antibakterielle Wirkung eines Glaspulvers nach Europ. Pharmakopoe (3. Auflage) für eine Glaszusammensetzung gemäß Ausführungsbeispiel 11 in Tabelle 1 mit einer Partikelgröße von 4 µm in einer wässerigen Suspension bei einer Konzentration von 0,01 Gew%. Das Glas wurde vor der Mahlung nicht getempert. | | | | | |
|---|---|---|---|---|---|
| | **E. coli** | **P. aeruginosa** | **S. aureus** | **C. albicans** | **A. niger** |
| **Start** | 290000 | 220000 | 250000 | 270000 | 280000 |
| **2 Tage** | 0 | 0 | 100 | <100 | 100000 |
| **7 Tage** | 0 | 0 | 0 | 0 | 30000 |
| **14 Tage** | 0 | 0 | 0 | 0 | 22000 |
| **21 Tage** | 0 | 0 | 0 | 0 | 14000 |
| **28 Tage** | 0 | 0 | 0 | 0 | 14000 |

Bei den vorangegangenen Tabellen 3 bis 5 bezeichnet der Startwert die Anzahl der zu Beginn der Messungen eingesetzten Bakterien. Liegt ein Wert von 0 vor, so sind keine Bakterien mehr messbar. Dies ist ein Nachweis für die antimikrobielle Wirkung des Glaspulvers.

Zum Nachweis der Freisetzung antimikrobieller Ionen über die Zeit wird in Tabelle 6 die Freisetzung von Ag-Ionen aus Glaspulver in eine wässerige Lösung angegeben.

In Tabelle 6 ist die Ionen-Freisetzung für Si, Na, B und Ag in mg/L unter kontinuierlicher Auslaugung nach 1 Stunde, nach 24 Stunden, nach 72 Stunden und nach 168 Stunden gemäß Ausführungsbeispiel 2 in Tabelle 1 und 2-c in Tabelle 2 mit einer Korngröße von 5µm, in einer wässerigen Suspension bei einer Konzentration von 1 Gew% angegeben.

**Tabelle 6:**

| **nach 1 Std. (mg/L)** | SiO₂ | Na₂O | B₂O₃ | Ag |
|---|---|---|---|---|
| Ausf.2 | 227 | 1283 | 6929 | 0,63 |
| Ausf.2-c | 781 | 3384 | 14019 | 6,1 |
| | | | | |

| **nach 24 Std. (mg/L)** | SiO₂ | Na₂O | B₂O₃ | Ag |
|---|---|---|---|---|
| Ausf.2 | 121 | 74 | 274 | 0,035 |
| Ausf.2-c | 164 | 37,6 | 36,1 | 0,44 |
| | | | | |

| **nach 72 Std. (mg/L)** | SiO₂ | Na₂O | B₂O₃ | Ag |
|---|---|---|---|---|
| Ausf.2 | 70,8 | 23,8 | 60,8 | 0,02 |
| Ausf.2-c | 61,3 | 4,6 | 4,70 | 0,36 |
| | | | | |

| **nach 168 Std. (mg/L)** | SiO₂ | Na₂O | B₂O₃ | Ag |
|---|---|---|---|---|
| Ausf.2 | 61,4 | 9,5 | 14,1 | 0,01 |
| Ausf.2-c | 16,3 | 2,62 | 2,89 | 0,3 |

Unter kontinuierlicher Auslaugung wird in dieser Anmeldung verstanden, dass nach z. B. 72 Std. Wasserdurchfluss, bei einem Glas gemäß Ausführungsbeispiel 2c beispielsweise noch 0,36 mg/l Silber freigesetzt werden, wie in Tabelle 6 angegeben.

Erkennbar ist, dass das entmischte Glas deutlich mehr Bor-, Natrium- und insbesondere Silber-Ionen als das nicht entmischte Glas am Anfang der Auslaugung freisetzt. Durch die niedrigere chemische Beständigkeit der borhaltigen Phase, wird die antimikrobielle Wirksamkeit erhöht.

Die borhaltige Phase ist die hochreaktive Phase des 2 Phasen Systems mit einer sehr schnellen Silberionen-Freisetzung, bzw. einer sehr starken kurzfristigen antimikrobiellen Wirkung. Die silikathaltige Phase sorgt durch ihre höhere chemische Beständigkeit für eine langsame Silberfreisetzung und die antimikrobielle Langzeit-Wirkung des Glases.

Als alternative Glaszusammensetzung können Zinkphosphatgläser als antimikrobielle Zusätze in Dentalmaterialien verwendet werden. Diese Glaszusammensetzungen sind in den Tabellen 8 und 9 angegeben:

**Tabelle 8:**

| Zusammensetzungen (Synthesewerte) [Gew%] von erfindungsgemäßen Glaszusammensetzungen | | | | | | | | | | | | | | | | | | |
|---|---|---|---|---|---|---|---|---|---|---|---|---|---|---|---|---|---|---|
| | A19 | A20 | A21 | A22 | A23 | A24 | A25 | A26 | A27 | A28 | A29 | A30 | A31 | A32 | A33 | A34 | A35 | A36 |
| P₂O₅ | 66,1 | 70 | 68 | 66,1 | 67 | 75 | 667,5 | 65,9 | 65,9 | 75 | 67 | 72 | 67 | 80 | 65,9 | 66,3 | 66 | 69 |
| SO₃ | | | | | | | | | | | | | | | | | | |
| B₂O₃ | | | | | | | | | | 1 | | | | | | 7,2 | 7 | |
| Al₂O₃ | 6,9 | 7 | 6,5 | 6,9 | 7 | 7 | 7 | 6,2 | 6,2 | 0 | 0 | 5 | 5 | 3 | 6,2 | 0,4 | | 6 |
| SiO₂ | | | | | | | | | | | | | | | 0.7 | 0,7 | 0,6 | 4 |
| Li₂O | | | | | | | | | | | | | | | | | | |
| Na₂O | 10 | 10,5 | 9 | 10 | 12,2 | 9,0 | 11 | | | | | | | 2,7 | | | | |
| K₂O | | | | | | | | | | | | | | | | | | |
| CaO | | | 8 | | 13 | | | 11,9 | 11,9 | | 11 | 20 | 8 | 5 | | 9,7 | 10 | 3 |
| MgO | | | | | | | | | | 8,6 | | | | | | 13,7 | 13,5 | 15 |
| SrO | | | | | | | | | | | | | | | | | | |
| BaO | | | | | | | | | | 13 | | | | | 11,90 | | | |
| ZnO | 16 | 12 | 8,5 | 10 | | 10 | 13,5 | 15 | 16 | 2 | 22 | 2 | 20 | 9 | 15 | | | |
| Ag₂O | 0,01 | 0,5 | | 0,5 | 0,8 | 2,0 | 1 | 1 | 0.5 | 0,5 | | 1 | | | 1 | 2 | 2 | 2 |
| CuO | | | | 0,01 | | | | | | | | | | | | | | |
| La₂O₃ | | | | | | | | | | | | | | 0,3 | | | | |
| ZrO₂ | | | | | | | | | | | | | | | | | 1 | 1 |

In Tabelle 9 ist die antimikrobielle Wirkung für das Ausführungsbeispiel 20 gemäß Tabelle 8 angegeben.

**Tabelle 9:**

| Antibakterielle Wirkung der Pulver nach Europ. Pharmakopoe (3. Auflage) in 0,001 Gew% wässeriger Lösung. Ausführungsbeispiel 25 Korngröße 4 µm: | | | | | |
|---|---|---|---|---|---|
| | **E. coli** | **P. aeruginosa** | **S. aureus** | **C. albicans** | **A. niger** |
| **Start** | 260000 | 350000 | 280000 | 360000 | 280000 |
| **2 Tage** | 0 | 0 | 0 | 0 | 0 |
| **7 Tage** | 0 | 0 | 0 | 0 | 0 |
| **14 Tage** | 0 | 0 | 0 | 0 | 0 |
| **21 Tage** | 0 | 0 | 0 | 0 | 0 |
| **28 Tage** | 0 | 0 | 0 | 0 | 0 |

Das Ausführungsbeispiel 25 besitzt in 1 %iger wässeriger Lösung einen pH-Wert von ca. 5,0.

In Tabelle 10 ist die antimikrobielle Wirkung für das Ausführungsbeispiel 26 gemäß Tabelle 8 angegeben. Es wurden 0,001 Gew% Glaspulver mit einer Partikelgröße von d50 = 4µm des Ausführungsbeispieles 26 in einer wässerigen Suspension gemessen.

**Tabelle 10: Antibakterielle Wirkung der Pulver nach Europ. Pharmakopoe (3. Auflage) in 0,001 Gew% wässeriger Suspension: Ausführungsbeispiel 26 gemäß Tabelle 8; Korngröße 4 µm**

| | **E.coli** | **P. aeruginosa** | **S. aureus** | **C. albicans** | **A. niger** |
|---|---|---|---|---|---|
| **Start** | 240000 | 340000 | 240000 | 330000 | 280000 |
| **2 Tage** | 0 | 0 | 0 | 55000 | 220000 |
| **7 Tage** | 0 | 0 | 0 | 40000 | 200000 |
| **14 Tage** | 0 | 0 | 0 | 0 | 0 |
| **21 Tage** | 0 | 0 | 0 | 0 | 0 |
| **28 Tage** | 0 | 0 | 0 | 0 | 0 |

Die Tabelle 11 ist die antimikrobielle Wirkung für das Ausführungsbeispiel 26 gemäß Tabelle 8 angegeben. Es wurden 0,01 Gew% Glaspulver mit einer Partikelgröße von d50 = 4µm des Ausführungsbeispieles 26 in einer wässerigen Suspension gemessen.

**Tabelle 11: Antibakterielle Wirkung der Pulver nach Europ. Pharmakopoe (3. Auflage) in 0,01 Gew% wässeriger Suspension: Ausführungsbeispiel 26 gemäß Tabelle 8: Korngröße 4 µm**

| | **E.coli** | **P. aeruginosa** | **S. aureus** | **C. albicans** | **A. niger** |
|---|---|---|---|---|---|
| **Start** | 240000 | 340000 | 240000 | 330000 | 280000 |
| **2 Tage** | 0 | 100 | 100 | 32000 | 260000 |
| **7 Tage** | 0 | 0 | 0 | 12000 | 240000 |
| **14 Tage** | 0 | 0 | 0 | 4400 | 200000 |
| **21 Tage** | 0 | 0 | 0 | 1000 | 140000 |
| **28 Tage** | 0 | 0 | 0 | 1000 | 140000 |

Als weitere besonders bevorzugte Glaszusammensetzung können Sulfophosphat-Gläser als Zusätze zu Dentalmaterialien eingesetzt werden. Derartige Gläser sind in den Tabellen 13 bis 15 angegeben.

**Tabelle 13:**

| Zusammensetzungen (Synthesewerte) [Gew%] von erfindungsgemäßen Glaszusammensetzungen | | | | | | | | |
|---|---|---|---|---|---|---|---|---|
| | Ausf. 37 | Ausf. 38 | Ausf.39 | Ausf. 40 | Ausf.41 | Ausf. 42 | Ausf. 43 | Ausf. 44 |
| P₂O₅ | 33,5 | 32,5 | 35 | 35,9 | 32,5 | 32,5 | 32,5 | 35 |
| SO₃ | 15 | 15 | 16 | 14 | 15 | 15 | 15 | 15 |
| B₂O₃ | | | | | | | | |
| Al₂O₃ | | | | | | | | |
| SiO₂ | | | | | | | | |
| Li₂O | | | | | | | | |
| Na₂O | 14,6 | 14,6 | 12,999 | 14,6 | 14,5 | 14,6 | 14,6 | 15 |
| K₂O | | | | | | | | |
| CaO | 3,3 | 3,3 | 2,4 | 35 | 11 | 3,3 | 3,3 | 10 |
| MgO | | | | | | | | |
| SrO | | | | | | | | |
| BaO | | | | | | | | |
| ZnO | 33,6 | 33,6 | 33,6 | | 26,5 | 33,6 | 33,6 | 25 |
| Ag₂O | | 1 | 0,0001 | 0,5 | 0,5 | 0,1 | | |
| CuO | | | | | | 0,3 | | |
| GeO₂ | | | | | | | | |
| TeO₂ | | | | | | | | |
| Cr₂O₃ | | | | | | 0,6 | | |
| J | | | | | | | 1 | |

**Tabelle 14:**

| Antibakterielle Wirkung der Pulver nach Europ. Pharmakopoe (3. Auflage) in 0,001 Gew% eines Glaspulvers gemäß Ausführungsbeispiel 38 mit einer mittleren Korngröße von 4 µm in wässeriger Suspension. | | | | | |
|---|---|---|---|---|---|
| | **E**. **coli** | **P. aeruginosa** | **S. aureus** | **C. albicans** | **A. niger** |
| **Start** | 270000 | 260000 | 260000 | 240000 | 240000 |
| **2 tage** | 0 | 0 | 0 | 0 | 160000 |
| **7 Tage** | 0 | 0 | 0 | 0 | 160000 |
| **14 Tage** | 0 | 0 | 0 | 0 | 140000 |
| **21 Tage** | 0 | 0 | 0 | 0 | 120000 |
| **28 Tage** | 0 | 0 | 0 | 0 | 10000 |

**Tabelle 15 zeigt die antimikrobielle Wirkung eines Glaspulvers gemäß Ausführungsbeispiel 38 in einer 0,1 Gew%-igen wässerigen Suspension.**

| | **E. coll** | **P. aeruginosa** | **S. aureus** | **C. albicans** | **A. niger** |
|---|---|---|---|---|---|
| **Start** | 250000 | 210000 | 240000 | 270000 | 280000 |
| **2 Tage** | 0 | 0 | 0 | 0 | 140000 |
| **7 Tage** | 0 | 0 | 0 | 0 | 20000 |
| **14 Tage** | 0 | 0 | 0 | 0 | 1500 |
| **21 Tage** | 0 | 0 | 0 | 0 | 100 |
| **28 Tage** | 0 | 0 | 0 | 0 | 100 |

Auch auf der Basis von Silicatgläsern können Zusätze von Dentalmaterialien erhalten werden. Derartige Gläser sind in Tabelle 16 angegeben.

**Tabelle 16:**

| Zusammensetzungen (Synthesewerte) [Gew%] von erfindungsgemäßen Glaszusammensetzungen | | | | | | | | | | | |
|---|---|---|---|---|---|---|---|---|---|---|---|
| Gew%. | A45 | A46 | A47 | A48 | A49 | A50 | A51 | A52 | A53 | A54 | A55 |
| SiO₂ | 71,00 | 45,00 | 44,50 | 35,00 | 34,90 | 44 | 60 | 59 | | 45 | 46,5 |
| Na₂O | 14,10 | 22,00 | 24,50 | 27,50 | 29,50 | 24,50 | 20 | 20 | 26,5 | 24,50 | 26,5 |
| CaO | 10,00 | 22,00 | 24,50 | 27,50 | 29,50 | 24,50 | 20 | 20 | 26,5 | 24,50 | 26,5 |
| P₂O₅ | - | 6,00 | 6,00 | 5,80 | 6,00 | 6,00 | - | - | - | 6,00 | - |
| Al₂O₃ | - | - | - | - | - | - | - | - | - | - | - |
| MgO | 4,70 | - | - | - | 0,10 | - | - | - | - | - | - |
| Ag₂O | 0,2 | - | 0,50 | 0.2 | - | 1 | - | 1 | - | - | 0,5 |
| AgJ | - | - | - | - | - | - | - | - | - | - | - |
| NaJ | - | - | - | - | - | - | - | - | - | - | - |
| TiO₂ | - | - | - | - | - | - | - | - | - | - | - |
| K₂O | - | - | - | - | - | - | - | - | - | - | - |
| ZnO | - | 5,0 | - | 4,0 | - | - | - | - | - | - | - |

In Tabelle 17 ist die Ionen- Freisetzung für Ag in mg/L unter stehender Auslaugung nach 1 Stunde und nach 24 Stunden gemäß Ausführungsbeispiel 12, 12c, 15, 19, 25, 26, 33 und 36 (siehe Tabelle 8) mit einer Korngröße von 5µm, in einer wässerigen Suspension und einer Konzentration von 1 Gew% angegeben.

**Tabelle 17:**

| **Silber Freisetzung in mg/L** | **1 Std.** | **24 Std.** |
|---|---|---|
| Ausf.12 | 9 | 10,8 |
| Ausf. 12-c | 32,9 | 68,6 |
| Ausf. 15 | 28,5 | 23,5 |
| Ausf. 19 | 28,5 | 50,5 |
| Ausf 25 | 2,3 | 11 |
| Ausf 26 | 2,9 | 17 |
| Ausf 33 | 2,2 | 6,4 |
| Ausf 36 | 7,89 | 47,4 |

Wie aus Tabelle 17 in Verbindung mit Tabelle 8 hervorgeht ist die Freisetzungsrate einstellbar durch die Glaszusammensetzung, durch den Grad der Keramisierung sowie durch die Silberkonzentration.

In Tabelle 18 sind weitere Zusammensetzungen in Gew% für Dentalglasfüller angegeben, die beispielsweise in Glasionomeren wie in Tabelle 19 beschrieben, eingesetzt werden können. Die Dentalglasfüller gemäß Tabelle 18 weisen alle bis auf das Ausführungsbeispiel 70 eine antimikrobielle Wirkung auf. In Tabelle 18 des weiteren angegeben ist die thermische Längenausdehung (CTE), die Brechzahl nD, die Transformationstemperatur Tg, die für Dentalfüller wichtige Radioopatizität für eine 2 mm dicke Probe, die Silberionenfreisetzung (Ag-Freisetzung) sowie das Onset OD.

**Tabelle 18: Zusammensetzungen für Dentalglasfüller**

| | A56 | A57 | A58 | A59 | A60 | A61 | A62 | A63 | A64 | A65 | A66 | A67 | A68 | A69 |
|---|---|---|---|---|---|---|---|---|---|---|---|---|---|---|
| SiO₂ | 60 | 50 | 99,5 | 45 | 30 | 30 | 30 | 50 | 50 | 54,5 | 50 | 60 | 30 | 5 |
| Al₂°O₃ | 20 | 20 | | 10 | 30 | 20 | 20 | 9,9 | 10 | 10 | 15 | 14 | 5 | |
| B₂°O₃ | | | | 10 | | | | 10 | 10 | 10 | 15 | 15 | 5 | 19,9 |
| ZnO | | | | | | 15 | 10 | | | | | | 10 | 20 |
| BaO | | | | 35 | | | | 30 | 30 | 25 | | | | |
| CaO | | | | | 10 | | | | | | | | 5 | |
| SrO | | | | | | | | | | | | | | |
| P₂° O₅ | | 5, | | | 9,5 | 3 | | | | | | | | |
| La₂°O₃ | | 10 | | | | | | | | | | | 5 | 35 |
| ZrO₂ | 5 | 5 | | | | | | | | | | | | |
| Li₂O | 5 | 5 | | | | | | | | | | | | |
| MgO | 5 | | | | | | | | | | | | | |
| K₂O | 1 | | | | | | | | | | | | | |
| Na₂O | | | | | | 2 | | | | | | | 5 | |
| ZrO₂ | | | | | | | | | | | | | 10 | |
| TiO₂ | | | | | | | | | | | | | | 5 |
| Nb₂°O₃ | | | | | | | | | | | | | | 10 |
| Ta₂°O₅ | 1 | 1 | | | | | | | | | | | | |
| WO₃ | | | | | | | | | | | | | | 5 |
| SrO | | | | | | 20 | 20 | | | | 20 | 15 | 25 | |
| Ag₂O | 1 | 2 | 0,5 | 1 | 0,5 | | 2 | 0,1 | 1 | 0,5 | 1 | 1 | | 0,1 |
| F | | | | 1 | 10 | 10 | 18 | | 1 | | | 2 | | |
| CTE (-30/+70) 10-6/K | c.a. 1 | c.a. 1 | 0,6 | | 10 | 7 | 7 | 5 | 5 | 4 | 4 | 3 | 8 | 6 |
| nD | 1,52 | 1,58 | 1,46 | 1,56 | 1,47 | 1,51 | 1,51 | 1,55 | 1,53 | 1,53 | 1,52 | 1,5 | 1,6 | 1,83 |
| Tg ISO 7884-8 | > 800 | > 800 | nicht besti mm bar | | 440 | 512 | 505 | 630 | 595 | 630 | 680 | 610 | 530 | 585 |
| Dichte (g/cm³) | 2,6 | 2.9 | 2,2 | | 2,6 | 3,1 | 3,1 | 3 | 2,9 | 2,8 | 2,6 | 2,46 | 3,42 | 4,55 |
| Radio Opazicität (ISO 4049) 2 mm glass dicke) | 1.5 (75 %) | 4.4( 220 %) | c.a. 5 (220 %) | c.a. 260 | c.a.1 (50 %) | c.a. 5 (250 %) | c.a. 5 (250 %) | 4,8 (240 %) | 4,8 (240 %) | 4.2 (210 %) | 4,2 (210 %) | c.a. 4 (200 %) | c.a. 6 (300 %) | c.a. 8 (400 %) |
| Ag Freisetzung (mg/L) nach 24 Std | | 0.031 | | | | | 0,042 | | | | 0,039 | | | |
| Onset OD (absolut | | 18,5 | | | | 16,8 | 18,2 | 5,7 | 6,8 | 6,8 | 15,9 | | | |
| Bewertung | | ○ | | | | ○ | ○ | □ | | ▲ | ○ | | | |

| | | | | | | | | | | | | | | |
|---|---|---|---|---|---|---|---|---|---|---|---|---|---|---|
| ▲....gering antibakteriell □.....sehr gering antibakteriell ○...... antibakteriell ◊.....keine Aktivität | | | | | | | | | | | | | | |

Nachfolgend sollen Beispiele für erfindungsgemäße Zusammensetzungen für Glasionomerzemente angegeben werden.

Die Angaben beziehen sich auf Gew% der Gesamtzusammensetzung.

| Glasionomer mit antimikrobiellem Glaspulver oder | flüssige Komponente |
|---|---|
| Glasionomer mit antimikrobieller Wirkung | |
| 50 Gew% | 50 Gew% Polyacrylsäure |
| | |
| 47,5 Gew% | 47,5 Gew% Polyacrylsäure |
| | 5 Gew% Weinsäure |
| | |
| 45 Gew% | 45 Gew% Polyacrylsäure |
| | 5 Gew% Weinsäure |
| | 5 Gew% CH₃OH |
| | |
| 75 Gew% | 15 Gew% Polyacrylsäure |
| | 10 Gew% Weinsäure |
| | |
| 64,3 Gew% | 25,7 Gew% Polyacrylsäure |
| | 10 Gew% Weinsäure |

Bei den oben angegebenen Zusammensetzungen können sämtlich hier genannten Glaspulver mit antimikrobieller Wirkung verwendet werden. Auch Mischungen von antimikrobiellen Glaspulvern mit herkömmlichen Glaspulvern sind möglich. Der Anteil an antimikrobiellem Glaspulver in der Mischung mit herkömmlichen Glasionomeren beträgt bevorzugt 0,5 bis 25 Gew%, bevorzugter 5 bis 15 Gew%. Alternativ kann das Glasionomere selbst ein antimikrobielles Glaspulver sein.

In nachfolgender Tabelle 19 sind Ausführungsbeispiele angegeben, bei denen ein Methacrylat-Monomer (ein sogenanntes Bis-GMA) mit einem nicht antimikrobiellen Dentalglasfüller A70 gemäss Tabelle 18 und einem antimikrobiellen Dentalglasfüller in der angegebenen Konzentration gemäß Tabelle 1, 2, 8, 13 und 18 zu einem Glasionomerzement gemischt wurde.

**Tabelle 19: Komponenten für einen Glasmonomerenzement in Gew% der Gesamtzusammensetzung**

| Monomerprobe | | | | | | | | |
|---|---|---|---|---|---|---|---|---|
| Bis GMA [%] | A70 [%] | AM-Pulver | | Transparenz [%] | Transluzenz [%] | Onset OD (absolute Werte) | Bewer- tung | Ag-Freisetzung nach 24 Std (mg/L) |
| | | Glas | [%] | | | | | |
| 100 | | | | 92,1 | 77,9 | 1,9 | ◇ | |
| 50 | 50 | | | 52,2 | 26,5 | 1,8 | ◇ | |
| 50 | 45 | A46 | 5 | 51,4 | 26,5 | 5,9 | ▲ | |
| 50 | 48 | A46 | 2 | 51,6 | 26,3 | 2,9 | • | |
| 50 | 20 | A21 | 30 | 51,5 | 28,8 | 15,3 | ○ | |
| 50 | 35 | A21 | 15 | 51,4 | 27,3 | 6,2 | ▲ | |
| 50 | 45 | A26 | 5 | 51,0 | 27,8 | | ○ | 0,029 |
| 50 | 48 | A26 | 2 | 51,7 | 27,4 | | ○ | 0,018 |
| 50 | 45 | A16 | 5 | 39,9 | 18,5 | 18,9 | ○ | 0,046 |
| 50 | 48 | A16 | 2 | 45,9 | 23,1 | 16,1 | ○ | 0,035 |
| 50 | 45 | A12-c | 5 | 33,2 | 16.5 | 17,7 | ○ | 0,041 |
| 50 | 48 | A12-c | 2 | 42,9 | 22,7 | 15,9 | ○ | 0,029 |
| 50 | 45 | A27 | 5 | 50,1 | 26,7 | 15,6 | ○ | |
| 50 | 48 | A27 | 2 | 49,1 | 25,0 | 14,9 | ○ | |
| 50 | 45 | A33 | 5 | 49,9 | 26,7 | 15,3 | ○ | |
| 50 | 48 | A33 | 2 | 51,4 | 27,0 | 6,2 | ▲ | |
| 50 | 45 | A17 | 5 | 40,2 | 17,4 | | | |
| 50 | 48 | A17 | 2 | 45,3 | 21,7 | | | |

| | | | | | | | | |
|---|---|---|---|---|---|---|---|---|
| ▲....geringe antibakteriell □.....sehr gering antibakteriell ○......antibakteriell •....sehr geringe Aktivität ◊.....keine Aktivität | | | | | | | | |

In Tabelle 20 ist die beobachtete Proliferation über 48 h gezeigt für ein Glaspulver mit einer Partikelgröße zwischen d50 von 4 µm und einer Glaszusammensetzung gemäß 1, das homogen in den angegebenen Konzentrationen (Gew%) in Zement eingebracht wurde.

Unter Onset OD wird die optische Dichte im umgebenden Nährmedium verstanden. Durch Proliferation (Bildung von Tochterzellen) und Abgabe der Zellen von der Oberfläche in das umgebende Nährmedium erfolgt eine Beeinträchtigung der Transmission des Nährmediums. Diese Absorption bei bestimmten Wellenlängen korreliert mit der antimikrobiellen Wirksamkeit der Oberfläche. Je höher der Onset OD Wert, desto stärker antimikrobiell wirksam ist die Oberfläche.

## Patentansprüche

1. Verwendung von Glaszusammensetzungen mit antimikrobieller und/oder desinfizierender Wirkung In Materialien zur Zahnrestauration, ausgenommen Implantate, Im Bereich der Zahnfoller, wobei der Zahnfüller ein Material, ausgewählt aus der nachfolgenden Gruppe, ist:
ein Glaslonomerenzement
ein Compomer,
wobei die Glaszusammensetzung die nachfolgenden Komponenten (In Gew.-% auf Oxidbasis) umfasst:
| | |
|---|---|
| SiO₂ | 0 - 99,5 Gew%.-% |
| P₂O₅ | 0 - 80 Gew.-% |
| SO₃ | 0 - 40 Gew.-% |
| B₂O₃ | 0 - 80 Gew.-% |
| Al₂O₃ | 0 - 30 Gew.-% |
| Li₂O | 0 - 30 Gew.-% |
| Na₂O | 0 - 40 Gew.-% |
| K₂O | 0 - 30 Gew.-% |
| CaO | 0 - 25 Gew.-% |
| MgO | 0 - 15 Gew.-% |
| SrO | 0 - 30 Gew.-% |
| BaO | 0 - 40 Gew.-% |
| ZnO | 0 - <15 Gew.-% |
| TiO₂ | 0 - 10 Gew.-% |
| ZrO₂ | 0 - 15 Gew.-% |
| CeO₂ | 0 - 10 Gew.-% |
| Ag₂O | 0,01 - 5 Gew.-% |
| F | 0 - 70 Gew.-% |
| J | 0 - 10 Gew.-% |
| Fe₂O₃ | 0 - 5 Gew.-% |
und gegebenenfalls Spurenelemente und/oder übliche Läutermittel In gängigen Mengen, wobei die Summe von SiO₂ + P₂O₅ + SO₃ + B₂O₃ + Al₂O₃ größer als 20 Gew.-% und maximal 99,5 Gew.-% und die Summe ZnO + Ag₂O + CuO + GeO₂ + TeO₂ + Cr₂O₃ > 0,01 Gew.-% ist;
oder
wobei die Glaszusammensetzung die nachfolgenden Komponenten (In Gew.-% auf Oxidbasis) umfasst:
| | |
|---|---|
| SiO₂ | 0 - 9,5 Gew.-%, bevorzugt 0 - 80 Gew.-% |
| P₂O₅ | 0 - 80 Gew.-% |
| SO₃ | 0 - 40 Gew.-% |
| B₂O₃ | 0 - 80 Gew.-% |
| Al₂O₃ | 0 - 30 Gew.-% |
| Li₂O | 0 - 30 Gew.-% |
| Na₂O | 0 - 40 Gew.-% |
| K₂O | 0 - 30 Gew.-% |
| CaO | 0 - 25 Gew.-% |
| MgO | 0 - 15 Gew.-% |
| SrO | 0 - 30 Gew.-% |
| BaO | 0 - 40 Gew.-% |
| ZnO | 0 - < 15 Gew.-%, bevorzugt 5 - < 15 Gew.-% |
| F | 0 - 65 Gew.-% |
| J | 0 - 10 Gew.-% |
| Fe₂O₃ | 0 - 5 Gew.-% |
| Ag₂O | 0,01 - 5 Gew.-% |
und gegebenenfalls Spurenelemente und/oder übliche Läutermittel in gängigen Mengen, wobei die Summe von SiO₂ + P₂O₆ + SO₃ + B₂O₃ + Al₂O₃ größer als 20 Gew.-% und maximal 99,5 Gew.-%, insbesondere maximal 80 Gew.-% ist
oder
wobei die Glaszusammensetzung eine Ionen-freisetzende Glaszusammensetzung ist, die nachfolgenden Komponenten umfasst (in Gew.-% auf Oxidbasis):
| | |
|---|---|
| SiO₂ | 40 - 80 Gew.-% |
| B₂O₃ | 5 - 40 Gew.-% |
| Al₂O₃ | 0 - 10 Gew.-% |
| P₂O₅ | 0 - 30 Gew.-% |
| Li₂O | 0 - 25 Gew.-% |
| Na₂O | 0 - 25 Gew.-% |
| K₂O | 0 - 25 Gew.-% |
| CaO | 0 - 25 Gew.-% |
| MgO | 0 - 15 Gew.-% |
| SrO | 0 - 15 Gew.-% |
| BaO | 0 - 15 Gew.-% |
| ZnO | 0 - < 15 Gew.-% |
| Ag₂O | 0,01 - 5 Gew.-% |
| CuO | 0 - 10 Gew.-% |
| GeO₂ | 0 - 10 Gew.-% |
| TeO₂ | 0 - 15 Gew.-% |
| Cr₂O₃ | 0 - 10 Gew.-% |
| J | 0 - 10 Gew.-% |
| F | 0 - 10 Gew.-% |
wobei die Summe ZnO + Ag₂O + CuO + GeO₂ + TeO₂ + Cr₂O₃ + J zwischen 5 und 70 Gew.-% liegt.

2. Verwendung nach Anspruch 1 in Beschichtungs-, Füll- oder Verblendmaterialien für keramische Dentalsuprastrukturen.

3. Verwendung gemäß einem der Ansprüche 1 oder 2,
**dadurch gekennzeichnet, dass**
die Glaszusammensetzung ZnO Im Bereich 0,25 bis < 15 Gew.-%, bevorzugt 2,5 bis 10 Gew.-% umfasst.

4. Verwendung gemäß einem der Ansprüche 1 bis 3,
**dadurch gekennzeichnet, dass**
die Glaszusammensetzung Ag₂O Im Bereich 0,05 bis 2 Gew.-%, besonders bevorzugt 0,5 bis 2 Gew.-% umfasst.

5. Verwendung gemäß einem der Ansprüche 1 bis 4,
dadurch gekennzelchnet, dass
die Summe BaO + SrO größer 10 Gew.-% Ist.

6. Verwendung einer Ionen-freisetzenden Glaszusammensetzung mit antimikrobieller Wirkung als Materialien zur Zahnrestauration, Insbesondere In Materialien zur Zahnfüllung, In Kombination mit Materialien zur Zahnfüllung, Insbesondere ausgewählt aus Glaslonomeren, Komposite, Compomere, wobei die Glaszusammensetzung
die nachfolgenden Komponenten umfasst (in Gew.-% auf Oxidbasis):
| | |
|---|---|
| P₂O₆ | > 66 - 80 Gew,-% |
| SO₃ | 0 - 40 Gew.-% |
| B₂O₃ | 0 - 1 Gew.-% |
| Al₂O₃ | > 6,2 - 10 Gew.-% |
| SiO₂ | 0 - 10 Gew.-% |
| Li₂O | 0 - 25 Gew.-% |
| Na₂O | > 8 - 20 Gew.-% |
| CaO | 0 - 25 Gew.-% |
| MgO | 0 - 15 Gew.-% |
| SrO | 0 - 15 Gew,-% |
| BaO | 0 - 15 Gew.-% |
| ZnO | 0 - < 15 Gew.-% |
| Ag₂O | 0 - 5 Gew.-% |
| CuO | 0 - 10 Gew.-% |
| GeO₂ | 0 - 10 Gew.-% |
| TeO₂ | 0 - 15 Gew.-% |
| Cr₂O₃ | 0 - 10 Gew.-% |
| J | 0 - 10 Gew.-% |
| F | 0 - 3 Gew.-% |
wobei die Summe ZnO + Ag₂O + CuO + GeO₂ + TeO₂ + Cr₂O₃ + J > 0,01 Gew.-% ist;
oder
wobei die Glaszusammensetzung
die nachfolgenden Komponenten umfasst (In Gew.-% auf Oxidbasis):
| | |
|---|---|
| P₂O₆ | > 66 - 80 Gew.-% |
| SO₃ | 0 - 40 Gew.-% |
| B₂O₃ | 0 - 1 Gew.-% |
| Al₂O₃ | 0 - 3,9 Gew.-% |
| SlO₂ | 0 - 10 Gew.-% |
| CaO | 0 - 25 Gew.-% |
| MgO | 0 - 15 Gew.-% |
| SrO | 0 - 15 Gew.-% |
| BaO | 0 - 15 Gew.-% |
| ZnO | 1 - < 15 Gew.-% |
| Ag₂O | 0 - 5 Gew.-% |
| CuO | 0 - 10 Gew.-% |
| GeO₂ | 0 - 10 Gew.-% |
| TeO₂ | 0 - 15 Gew.-% |
| Cr₂O₃ | 0 - 10 Gew.-% |
| J | 0 - 10 Gew.-% |
| F | 0 - 3 Gew.-% |
wobei die Summe ZnO + Ag₂O + CuO + GeO₂ + TeO₂ + Cr₂O₃ + J > 1 Gew.-% Ist;
oder
wobei die Glaszusammensetzung
die nachfolgenden Komponenten umfasst (in Gew.-% auf Oxidbasis):
| | |
|---|---|
| P₂O₆ | > 45 - 90 Gew.-% |
| B₂O₃ | 0 - 60 Gew.-% |
| SiO₂ | 0 - 40 Gew.-% |
| Al₂O₃ | 0 - 20 Gew.-% |
| SO₃ | 0 - 30 Gew.-% |
| Li₂O | 0 - 0,1 Gew.-% |
| Na₂O | 0 - 0,1 Gew.-% |
| K₂O | 0 - 0,1 Gew.-% |
| CaO | 0 - 40 Gew.-% |
| MgO | 0 - 40 Gew.-% |
| SrO | 0 - 15 Gew.-% |
| BaO | 0 - 40 Gew.-% |
| ZnO | 0 - < 15 Gew.-% |
| Ag₂O | 0 - 5 Gew.-% |
| CuO | 0 - 15 Gew.-% |
| Cr₂O₃ | 0 - 10 Gew.-% |
| J | 0 - 10 Gew.-% |
| TeO₂ | 0 - 10 Gew.-% |
| GeO₂ | 0 - 10 Gew.-% |
| TIO₂ | 0 - 10 Gew.-% |
| ZrO₂ | 0 - 10 Gew.-% |
| La₂O₃ | 0 - 10 Gew.-% |
| Nb₂O₃ | 0 - 5 Gew.-% |
| CeO₂ | 0 - 5 Gew.-% |
| Fe₂O₃ | 0 - 5 Gew.-% |
| WO₃ | 0 - 5 Gew.-% |
| Bi₂O₃ | 0 - 5 Gew.-% |
| MoO₃ | 0 - 5 Gew.-% |
wobei die Summe ZnO + Ag₂O + CuO + GeO₂ + TeO₂ + Cr₂O₃ + J > 0,001 Gew.-% Ist;
und
wobei die obigen Glaszusammensetzungen
ZnO Im Bereich 0,25 bis < 15 Gew.-%, bevorzugt 2,5 bis 10 Gew.-% und/oder
Ag₂O Im Bereich 0,05 bis 2 Gew.-%, besonders bevorzugt 0,5 bis 2 Gew.-%, umfassen.

7. Verwendung gemäß Anspruch 6,
**dadurch gekennzeichnet, dass**
die Glaszusammensetzung BaO und SrO enthält und die Summe BaO + SrO größer 10 Gew.-% Ist.

8. Verwendung einer Ionen-freisetzenden Glaszusammensetzung gemäß Anspruch 6 bis 7,
**dadurch gekennzeichnet, dass**
In der Glaszusammensetzung mindestens zwei Glasphasen ausgebildet werden.

9. Verwendung einer ionen-freisetzenden Glaszusammensetzung gemäß Anspruch 8,
**dadurch gekennzeichnet, dass**
In der Glaszusammensetzung mindestens zwei Glasphasen unterschiedliche Zusammensetzungen aufweisen.

10. Verwendung einer Ionen-freisetzenden Glaszusammensetzung gemäß einem der Ansprüche 8 bis 9,
**dadurch gekennzeichnet, dass**
die Glaszusammensetzung eine Borosilicatglaszusammensetzung ist.

11. Verfahren zur Herstellung einer Ionen-freisetzenden Glaszusammensetzung
gemäß einem der Ansprüche 8 bis 10,
**dadurch gekennzeichnet, dass**
die mindestens zwei Phasen durch Tempern In einem Temperaturbereich Tg ≤ T ≤ Tg + 300°C erhalten werden, wobei Tg die Transformationstemperatur des Glases Ist.

12. Verwendung einer Ionen-freisetzenden antimikrobiellen Glaszusammensetzung gemäß einem der Ansprüche 6 bis 10 für einen Ionen-freisetzenden Glasionomerzement für Dentalanwendungen, weiterhin umfassend:
ein Polymer, das freie Carbonsäuregruppen enthält.

13. Verwendung gemäß Anspruch 12,
**dadurch gekennzeichnet, dass**
1 - 90 Gew.-% der
Gesamtzusammensetzung eine Ionen freisetzende Glas-/Glaskeramikzusammensetzung ist, wobei die ionen-freisetzende Glaszusammensetzung eine Ionen-freisetzende antimikrobielle Glaszusammensetzung oder eine Ionen-freisetzende Glaskeramik umfasst oder eine Mischung aus einer ionenfreisetzenden Glaslonomerenzusammensetzung mit einer ionen-freisetzenden antimikrobiellen Glaszusammensetzung oder einer ionen-freisetzenden Glaskeramik Ist.

14. Verwendung gemäß einem der Ansprüche 12 bis 13,
**dadurch gekennzeichnet, dass**
das Verhältnis von antimikrobieller Glaszusammensetzung/Glaslonomerzement und/oder Zahnfüller > 0,001 ist.

15. Verwendung gemäß einem der Ansprüche 12 bis 14,
**dadurch gekennzeichnet, dass**
das Verhältnis von antimikrobieller Glaszusammensetzung/Glasionomerzement und/oder Zahnfüller < 200, bevorzugter kleiner als 100, ganz bevorzugt kleiner als 10 Ist.

16. Verwendung einer Ionen-freisetzende antimikrobielle Glaszusammensetzung gemäß einem der Ansprüche 6 bis 10 als Beschichtungs- oder Verblendungsmaterial für keramische Dentalsuprastrukturen, weiterhin umfassend ein Grundmaterial, bevorzugt einen Zahnfüller, Insbesondere ausgewählt aus:
einem Glasionomerzement,
einem Compomer.

## Claims

1. Use of glass compositions with antimicrobial and/or disinfectant effect in materials used for restoring teeth, excluding implants, in the field of tooth fillers, wherein the tooth filler is a material chosen from the following group:
a glass ionomer cement;
a compomer;
wherein the glass composition comprises the following components (in % by weight on the basis of oxide):
| | |
|---|---|
| SiO₂ | 0 - 99.5% by weight |
| P₂O₅ | 0 - 80% by weight |
| SO₃ | 0 - 40% by weight |
| B₂O₃ | 0 - 80% by weight |
| Al₂O₃ | 0 - 30% by weight |
| Li₂O | 0 - 30% by weight |
| Na₂O | 0 - 40% by weight |
| K₂O | 0 - 30% by weight |
| CaO | 0 - 25% by weight |
| MgO | 0 - 15% by weight |
| SrO | 0 - 30% by weight |
| BaO | 0 - 40% by weight |
| ZnO | 0 - < 15% by weight |
| TiO₂ | 0 - 10% by weight |
| ZrO₂ | 0 - 15% by weight |
| CeO₂ | 0 - 10% by weight |
| Ag₂O | 0.01 - 5% by weight |
| F | 0 - 70% by weight |
| J | 0 - 10% by weight |
| Fe₂O₃ | 0 - 5% by weight, |
and optionally trace elements and/or the usual refining agents in the usual quantities, wherein the sum total of SiO₂ + P₂O₅ + SO₃ + B₂O₃ + Al₂O₃ is greater than 20% by weight and a maximum of 99.5% by weight, and the sum total of ZnO + Ag₂O + CuO + GeO₂ + TeO₂ + Cr₂O₃ is > 0.01% by weight,
or
wherein the glass composition comprises the following components (in % by weight on the basis of oxide):
| | |
|---|---|
| SiO₂ | 0 - 99.5% by weight, |
| preferably 0 - 80% by weight | |
| P₂O₅ | 0 - 80% by weight |
| SO₃ | 0 - 40% by weight |
| B₂O₃ | 0 - 80% by weight |
| Al₂O₃ | 0 - 30% by weight |
| Li₂O | 0 - 30% by weight |
| Na₂O | 0 - 40% by weight |
| K₂O | 0 - 30% by weight |
| CaO | 0 - 25% by weight |
| MgO | 0 - 15% by weight |
| SrO | 0 - 30% by weight |
| BaO | 0 - 40% by weight |
| ZnO | 0 - < 15% by weight, |
| preferably 5 - < 15% by weight | |
| F | 0 - 65% by weight |
| J | 0 - 10% by weight |
| Fe₂O₃ | 0 - 5% by weight |
| Ag₂O | 0.01 - 5% by weight, |
and optionally trace elements and/or the usual refining agents in the usual quantities, wherein the sum total of SiO₂ + P₂O₅ + SO₃ + B₂O₃ + Al₂O₃ is greater than 20% by weight and a maximum of 99.5% by weight, especially a maximum of 80% by weight,
or
wherein the glass composition is an ion-releasing glass composition which comprises the following components (in % by weight on the basis of oxide):
| | |
|---|---|
| SiO₂ | 40 - 80% by weight |
| B₂O₃ | 5 - 40% by weight |
| Al₂O₃ | 0 - 10% by weight |
| P₂O₅ | 0 - 30% by weight |
| Li₂O | 0 - 25% by weight |
| Na₂O | 0 - 25% by weight |
| K₂O | 0 - 25% by weight |
| CaO | 0 - 25% by weight |
| MgO | 0 - 15% by weight |
| SrO | 0 - 15% by weight |
| BaO | 0 - 15% by weight |
| ZnO | 0 - < 15% by weight |
| Ag₂O | 0.01 - 5% by weight |
| CuO | 0 - 10% by weight |
| GeO₂ | 0 - 10% by weight |
| TeO₂ | 0 - 15% by weight |
| Cr₂O₃ | 0 - 10% by weight, |
| J | 0 - 10% by weight |
| F | 0 - 10% by weight, |
wherein the sum total of ZnO + Ag₂O + CuO + GeO₂ + TeO₂ + Cr₂O₃ + J is between 5 and 70% by weight.

2. The use according to claim 1 in coating, filling or veneering materials for ceramic dental superstructures.

3. The use according to one of the claims 1 or 2, **characterized in that** the glass composition comprises ZnO in the range of 0.25 to < 15% by weight, preferably 2.5 to 10% by weight.

4. The use according to one of the claims 1 to 3, **characterized in that** the glass composition comprises Ag₂O in the range of 0.05 to 2% by weight, especially preferably 0.5 to 2% by weight.

5. The use according to one of the claims 1 to 4, **characterized in that** the sum total of BaO + SrO is greater than 10% by weight.

6. The use of an ion-releasing glass composition with antimicrobial effect as materials for restoring teeth, especially in materials for tooth filling, in combination with materials for tooth filling, especially chosen from glass ionomers, compositions, compomers, wherein the glass composition comprises the following components (in % by weight on the basis of oxide):
| | |
|---|---|
| P₂O₅ | > 66 - 80% by weight |
| SO₃ | 0 - 40% by weight |
| B₂O₃ | 0 - 1% by weight |
| Al₂O₃ | > 6.2 - 10% by weight |
| SiO₂ | 0 - 10% by weight |
| Li₂O | 0 - 25% by weight |
| Na₂O | > 9 - 20% by weight |
| CaO | 0 - 25% by weight |
| MgO | 0 - 15% by weight |
| SrO | 0 - 15% by weight |
| BaO | 0 - 15% by weight |
| ZnO | 0 - < 15% by weight |
| Ag₂O | 0 - 5% by weight |
| CuO | 0 - 10 % by weight |
| GeO₂ | 0 - 10% by weight |
| TeO₂ | 0 - 15% by weight |
| Cr₂O₃ | 0 - 10% by weight |
| J | 0 - 10% by weight |
| F | 0 - 3% by weight |
wherein the sum total of ZnO + Ag₂O + CuO + GeO₂ + TeO₂ + Cr₂O₃ + J is > 0.01% by weight,
or
wherein the glass composition comprises the following components (in % by weight on the basis of oxide):
| | |
|---|---|
| P₂O₅ | > 66 - 80% by weight |
| SO₃ | 0 - 40% by weight |
| B₂O₃ | 0 - 1 % by weight |
| Al₂O₃ | 0 - 3.9% by weight |
| SiO₂ | 0 - 10% by weight |
| CaO | 0 - 25% by weight |
| MgO | 0 - 15% by weight |
| SrO | 0 - 15% by weight |
| BaO | 0 - 15% by weight |
| ZnO | 1 - < 15% by weight |
| Ag₂O | 0 - 5% by weight |
| CuO | 0 - 10 % by weight |
| GeO₂ | 0 - 10% by weight |
| TeO₂ | 0 - 15% by weight |
| Cr₂O₃ | 0 - 10% by weight |
| J | 0 - 10% by weight |
| F | 0 - 3% by weight |
wherein the sum total of ZnO + Ag₂O + CuO + GeO₂ + TeO₂ + Cr₂O₃ + J is > 1 % by weight,
or
wherein the glass composition comprises the following components (in % by weight on the basis of oxide):
| | |
|---|---|
| P₂O₅ | > 45 - 90% by weight |
| B₂O₃ | 0 - 60% by weight |
| SiO₂ | 0 - 40% by weight |
| Al₂O₃ | 0 - 20% by weight |
| SO₃ | 0 - 30% by weight |
| Li₂O | 0 - 0.1% by weight |
| Na₂O | 0 - 0.1% by weight |
| K₂O | 0 - 0.1% by weight |
| CaO | 0 - 40% by weight |
| MgO | 0 - 40% by weight |
| SrO | 0 - 15% by weight |
| BaO | 0 - 40% by weight |
| ZnO | 0 - < 15% by weight |
| Ag₂O | 0 - 5% by weight |
| CuO | 0 - 15% by weight |
| Cr₂O₃ | 0 - 10% by weight |
| J | 0 - 10% by weight |
| TeO₂ | 0 - 10% by weight |
| GeO₂ | 0 - 10% by weight |
| TiO₂ | 0 - 10% by weight |
| ZrO₂ | 0 - 10% by weight |
| La₂O₃ | 0 - 10% by weight |
| Nb₂O₃ | 0 - 5% by weight |
| CeO₂ | 0 - 5% by weight |
| Fe₂O₃ | 0 - 5% by weight |
| WO₃ | 0 - 5% by weight |
| Bi₂O₃ | 0 - 5% by weight |
| MoO₃ | 0 - 5% by weight |
wherein the sum total of ZnO + Ag₂O + CuO + GeO₂ + TeO₂ + Cr₂O₃ + J is > 0.001% by weight,
and
wherein the above glass compositions comprise ZnO in the range of 0.25 to < 15% by weight, preferably 2.5 to 10% by weight,
and/or
Ag₂O in the range of 0.05 to 2% by weight, especially preferably 0.5 to 2% by weight.

7. The use according to claim 6, **characterized in that** the glass composition contains BaO and SrO and the sum total of BaO + SrO is greater than 10% by weight.

8. The use of an ion-releasing glass composition according to claim 6 to 7, **characterized in that** at least two glass phases are formed in the glass composition.

9. The use of an ion-releasing glass composition according to claim 8, **characterized in that** at least two glass phases comprise different compositions in the glass composition.

10. The use of an ion-releasing glass composition according to one of the claims 8 to 9, **characterized in that** the glass composition is a borosilicate glass composition.

11. A method for producing an ion-releasing glass composition according to one of the claims 8 to 10, **characterized in that** the at least two phases are obtained by tempering in a temperature range of Tg ≤ T ≤ Tg + 300°C, wherein Tg is the transformation temperature of the glass.

12. The use of an ion-releasing antimicrobial glass composition according to one of the claims 6 to 10 for an ion-releasing glass ionomer cement for dental applications, further comprising:
a polymer containing free carboxylic acid groups.

13. The use according to claim 12, **characterized in that** 1 to 90% by weight of the total composition is an ion-releasing glass/glass ceramic composition, wherein the ion-releasing glass composition comprises an ion-releasing antimicrobial glass composition or an ion-releasing ceramic glass, or is a mixture of an ion-releasing glass ionomer composition with an ion-releasing antimicrobial glass composition or an ion-releasing ceramic glass.

14. The use according to one of the claims 12 to 13, **characterized in that** the ratio of antimicrobial glass composition/glass ionomer cement and/or tooth filler is > 0.001.

15. The use according to one of the claims 12 to 14, **characterized in that** the ratio of antimicrobial glass composition/glass ionomer cement and/or tooth filler is < 200, preferably less than 100, more preferably less than 10.

16. The use of an ion-releasing antimicrobial glass composition according to one of the claims 6 to 10 as a coating or veneering material for ceramic dental superstructures, further comprising a base material, preferably a tooth filler, especially selected from:
a glass ionomer cement,
a compomer.

## Revendications

1. Utilisation de compositions de verre ayant un effet antimicrobien et/ou désinfectant dans des matériaux pour la restauration dentaire, à l'exception des implants, dans le domaine des comblements dentaires, dans laquelle le comblement dentaire est un matériau choisi dans le groupe suivant:
un ciment au verre ionomère,
un compomère,
laquelle composition de verre contient les composants suivants (en % en poids sur la base de l'oxyde):
| | |
|---|---|
| SiO₂ | de 0 à 99,5 % en poids |
| P₂O₅ | de 0 à 80 % en poids |
| SO₃ | de 0 à 40 % en poids |
| B₂O₃ | de 0 à 80 % en poids |
| Al₂O₃ | de 0 à 30 % en poids |
| Li₂O | de 0 à 30 % en poids |
| Na₂O | de 0 à 40 % en poids |
| K₂O | de 0 à 30 % en poids |
| CaO | de 0 à 25 % en poids |
| MgO | de 0 à 15 % en poids |
| SrO | de 0 à 30 % en poids |
| BaO | de 0 à 40 % en poids |
| ZnO | de 0 à <15 % en poids |
| TiO₂ | de 0 à 10 % en poids |
| ZrO₂ | de 0 à 15 % en poids |
| CeO₂ | de 0 à 10 % en poids |
| Ag₂O | de 0,01 à 5 % en poids |
| F | de 0 à 70 % en poids |
| I | de 0 à 10 % en poids |
| Fe₂O₃ | de 0 à 5 % en poids |
et éventuellement des éléments traces et/ou des agents d'affinage usuels dans les quantités habituelles, la somme SiO₂ + P₂O₅ + SO₃ + B₂O₃ + Al₂O₅ représentant plus de 20 % en poids et au maximum 99,5 % en poids et la somme ZnO + Ag₂O + CuO + GeO₂ + TeO₂ + Cr₂O₃ représentant plus de 0,01 % en poids ;
ou
laquelle composition de verre contient les composants suivants (en % en poids sur la base de l'oxyde):
| | |
|---|---|
| SiO₂ | de 0 à 99,5 % en poids, de préférence de 0 à 80 % en poids |
| P₂O₅ | de 0 à 80 % en poids |
| SO₃ | de 0 à 40 % en poids |
| B₂O₃ | de 0 à 80 % en poids |
| Al₂O₃ | de 0 à 30 % en poids |
| Li₂O | de 0 à 30 % en poids |
| Na₂O | de 0 à 40 % en poids |
| K₂O | de 0 à 30 % en poids |
| CaO | de 0 à 25 % en poids |
| MgO | de 0 à 15 % en poids |
| SrO | de 0 à 30 % en poids |
| BaO | de 0 à 40 % en poids |
| ZnO | de 0 à < 15 % en poids, de préférence de 5 à < 15 % en poids |
| F | de 0 à 65 % en poids |
| I | de 0 à 10 % en poids |
| Fe₂O₃ | de 0 à 5 % en poids |
| Ag₂O | de 0,01 à 5 % en poids |
et éventuellement des éléments traces et/ou des agents d'affinage usuels dans les quantités habituelles, la somme SiO₂ + P₂O₅ + SO₃ + B₂O₃ + Al₂O₃ représentant plus de 20 % en poids et au maximum 99,5 % en poids, en particulier 80 % en poids au maximum;
ou
laquelle composition de verre est une composition de verre libérant des ions, qui contient les composants suivants (en % en poids sur la base de l'oxyde):
| | |
|---|---|
| SiO₂ | de 40 à 80 % en poids |
| B₂O₃ | de 5 à 40 % en poids |
| Al₂O₃ | de 0 à 10 % en poids |
| P₂O₅ | de 0 à 30 % en poids |
| Li₂O | de 0 à 25 % en poids |
| Na₂O | de 0 à 25 % en poids |
| K₂O | de 0 à 25 % en poids |
| CaO | de 0 à 25 % en poids |
| MgO | de 0 à 15 % en poids |
| SrO | de 0 à 15 % en poids |
| BaO | de 0 à 15 % en poids |
| ZnO | de 0 à < 15 % en poids |
| Ag₂O | de 0,01 à 5 % en poids |
| CuO | de 0 à 10 % en poids |
| GeO₂ | de 0 à 10 % en poids |
| TeO₂ | de 0 à 15 % en poids |
| Cr₂O₃ | de 0 à 10 % en poids |
| I | de 0 à 10 % en poids |
| F | de 0 à 10 % en poids |
la somme ZnO + Ag₂O + CuO + GeO₂ + TeO₂ + Cr₂O₃ + I représentant entre 5 et 70 % en poids.

2. Utilisation selon la revendication 1 dans des matériaux de revêtement, de comblement ou d'habillage pour des superstructures dentaires en céramique.

3. Utilisation selon l'une des revendications 1 ou 2, **caractérisée en ce que** la composition de verre contient du ZnO à raison de 0,25 à < 15 % en poids, de préférence de 2,5 à 10 % en poids.

4. Utilisation selon l'une des revendications 1 à 3, **caractérisée en ce que** la composition de verre contient de l'Ag₂O à raison de 0,05 à 2 % en poids, en particulier de 0,5 à 2 % en poids.

5. Utilisation selon l'une des revendications 1 à 4, **caractérisée en ce que** la somme BaO + SrO représente plus de 10 % en poids.

6. Utilisation d'une composition de verre libérant des ions ayant un effet antimicrobien et/ou désinfectant dans des matériaux pour la restauration dentaire, en particulier dans des matériaux de comblement dentaire, en combinaison avec des matériaux de comblement dentaire choisis en particulier parmi les verres ionomères, les composites, les compomères, la composition de verre contenant les composants suivants (en % en poids sur la base de l'oxyde) :
| | |
|---|---|
| P₂O₅ | de > 66 à 80 % en poids |
| SO₃ | de 0 à 40 % en poids |
| B₂O₃ | de 0 à 1 % en poids |
| Al₂O₃ | de > 6,2 à 10 % en poids |
| SiO2 | 0 à 10 % en poids |
| Li₂O | de 0 à 25 % en poids |
| Na₂O | de >9 à 20 % en poids |
| CaO | de 0 à 25 % en poids |
| MgO | de 0 à 15 % en poids |
| SrO | de 0 à 15 % en poids |
| BaO | de 0 à 15 % en poids |
| ZnO | de 0 à < 15 % en poids |
| Ag₂O | de 0 à 5 % en poids |
| CuO | de 0 à 10 % en poids |
| GeO₂ | de 0 à 10 % en poids |
| TeO₂ | de 0 à 15 % en poids |
| Cr₂O₃ | de 0 à 10 % en poids |
| I | de 0 à 10 % en poids |
| F | de 0 à 3 % en poids |
la somme ZnO + Ag₂O + CuO + GeO₂ + TeO₂ + Cr₂O₃ + I représentant plus de 0,01 % en poids;
ou
la composition de verre contenant les composants suivants (en % en poids sur la base de l'oxyde):
| | |
|---|---|
| P₂O₅ | de > 66 à 80 % en poids |
| SO₃ | de 0 à 40 % en poids |
| B₂O₃ | de 0 à 1 % en poids |
| Al₂O₃ | de 0 à 3,9 % en poids |
| SiO2 | de 0 à 10 % en poids |
| CaO | de 0 à 25 % en poids |
| MgO | de 0 à 15 % en poids |
| SrO | de 0 à 15 % en poids |
| BaO | de 0 à 15 % en poids |
| ZnO | de 1 à < 15 % en poids |
| Ag₂O | de 0 à 5 % en poids |
| CuO | de 0 à 10 % en poids |
| GeO₂ | de 0 à 10 % en poids |
| TeO₂ | de 0 à 15 % en poids |
| Cr₂O₃ | de 0 à 10 % en poids |
| I | de 0 à 10 % en poids |
| F | de 0 à 3 % en poids |
la somme ZnO + Ag₂O + CuO + GeO₂ + TeO₂ + Cr₂O₃ + I représentant plus de 1 % en poids;
ou
la composition de verre contenant les composants suivants (en % en poids sur la base de l'oxyde):
| | |
|---|---|
| P₂O₅ | de >45 à 90 % en poids |
| B₂O₃ | de 0 à 60 % en poids |
| SiO₂ | de 0 à 40 % en poids |
| Al₂O₃ | de 0 à 20 % en poids |
| SO₃ | de 0 à 30 % en poids |
| Li₂O | de 0 à 0,1 % en poids |
| Na₂O | de 0 à 0,1 % en poids |
| K₂O | de 0 à 0,1 % en poids |
| CaO | de 0 à 40 % en poids |
| MgO | de 0 à 40 % en poids |
| SrO | de 0 à 15 % en poids |
| BaO | de 0 à 40 % en poids |
| ZnO | de 0 à < 15 % en poids |
| Ag₂O | de 0 à 5 % en poids |
| CuO | de 0 à 15 % en poids |
| Cr₂O₃ | de 0 à 10 % en poids |
| I | de 0 à 10 % en poids |
| TeO₂ | de 0 à 10 % en poids |
| GeO₂ | de 0 à 10 % en poids |
| TiO₂ | de 0 à 10 % en poids |
| ZrO₂ | de 0 à 10 % en poids |
| La₂O₃ | de 0 à 10 % en poids |
| Nb₂O₃ | de 0 à 5 % en poids |
| CeO₂ | de 0 à 5 % en poids |
| Fe₂O₃ | de 0 à 5 % en poids |
| WO₃ | de 0 à 5 % en poids |
| Bi₂O₃ | de 0 à 5 % en poids |
| MoO₃ | de 0 à 5 % en poids |
la somme ZnO + Ag₂O + CuO + GeO₂ + TeO₂ + Cr₂O₃ + I représentant plus de 0,001 % en poids ;
et
les compositions de verre ci-dessus contenant
entre 0,25 et < 15 % en poids de ZnO, de préférence entre 2,5 et 10 % en poids
et/ou
entre 0,05 et 2 % en poids d'Ag₂O, en particulier entre 0,5 et 2 % en poids.

7. Utilisation selon la revendication 6, **caractérisée en ce que** la composition de verre contient du BaO et du SrO et la somme BaO + SrO représente plus de 10 % en poids.

8. Utilisation d'une composition de verre libérant des ions selon les revendications 6 à 7, **caractérisée en ce qu'**au moins deux phases vitreuses sont formées dans la composition de verre.

9. Utilisation d'une composition de verre libérant des ions selon la revendication 8, **caractérisée en ce qu'**au moins deux phases vitreuses dans la composition de verre présentent des compositions différentes.

10. Utilisation d'une composition de verre libérant des ions selon l'une des revendications 8 à 9, **caractérisée en ce que** la composition de verre est une composition de verre borosilicate.

11. Procédé pour la fabrication d'une composition de verre libérant des ions selon l'une des revendications 8 à 10, **caractérisé en ce que** les au moins deux phases sont obtenues par trempage dans une plage de température Tg ≤ T ≤ Tg + 300°C, où Tg est la température de transformation du verre.

12. Utilisation d'une composition de verre antimicrobienne libérant des ions selon l'une des revendications 6 à 10 pour un ciment au verre ionomère libérant des ions pour des applications dentaires, comprenant en outre un polymère qui contient des groupements d'acide carboxylique libres.

13. Utilisation selon la revendication 12, **caractérisée en ce qu'**entre 1 et 90 % en poids de la composition totale est une composition de verre ou de vitrocéramique libérant des ions, la composition de verre libérant des ions étant une composition de verre libérant des ions antimicrobienne ou un mélange d'une composition de verre ionomère libérant des ions avec une composition de verre libérant des ions antimicrobienne ou avec une vitrocéramique libérant des ions.

14. Utilisation selon l'une des revendications 12 à 13, **caractérisée en ce que** le rapport entre la composition de verre antimicrobienne et le ciment au verre ionomère et/ou le comblement dentaire est supérieur à 0,001.

15. Utilisation selon l'une des revendications 12 à 14, **caractérisée en ce que** le rapport entre la composition de verre antimicrobienne et le ciment au verre ionomère et/ou le comblement dentaire est inférieur à 200, de préférence inférieur à 100, en particulier inférieur à 10.

16. Utilisation d'une composition de verre antimicrobienne libérant des ions selon l'une selon l'une des revendications 6 à 10 comme matériau d'enduction ou d'habillage pour des superstructures dentaires en céramique, contenant en outre un matériau de base, de préférence un produit de comblement dentaire, en particulier choisi parmi:
un ciment au verre ionomère un compomère.
